# EUROPEAN PATENT SPECIFICATION

(11) **EP 2 575 685 B1**
(45) Date of publication and mention of the grant of the patent: **13.02.2019**
(21) Application number: 11792047.0
(22) Date of filing: 06.06.2011
(51) Int. Cl.: A61F 2/24

(54) **APPARATUS FOR GUIDE-WIRE BASED ADVANCEMENT OF A ROTATION ASSEMBLY**
VORRICHTUNG FÜR FÜHRUNGSDRAHT-BASIERTE WEITERENTWICKLUNG EINER ROTATIONSANORDNUNG
APPAREIL POUR L'AVANCEMENT BASÉ SUR UN FIL-GUIDE D'UN ENSEMBLE DE ROTATION

(30) Priority: 07.06.2010 US 795026; 07.06.2010 US 795192
(43) Date of publication of application: 10.04.2013
(60) Divisional application: 18192476.2
(73) Proprietor: Valtech Cardio, Ltd., 60376 Or Yehuda (IL)
(72) Inventor: MILLER, Eran, 76803 Moshav Beit Elazari (IL); CABIRI, Oz, 71799 Macabim-Reut (IL); REICH, Tal, 30500 Binyamina (IL); SHEPS, Tal, 54014 Givat Shmuel (IL)
(74) Representative: Eisenführ Speiser
(86) International application number: PCT/IL2011/000446
(87) International publication number: WO 2011/154942

(56) References cited:
- WO-A2-03/105667
- US-A1- 2003 105 519
- US-A1- 2007 118 151
- US-A1- 2007 213 582
- US-A1- 2010 023 118
- US-A1- 2010 042 147

## Description

### FIELD OF THE INVENTION

The present invention relates in general to valve and chordeae tendineae repair. More specifically, the present invention relates to repair of an atrioventricular valve and associated chordeae tendineae of a patient.

### BACKGROUND

Ischemic heart disease causes mitral regurgitation by the combination of ischemic dysfunction of the papillary muscles, and the dilatation of the left ventricle that is present in ischemic heart disease, with the subsequent displacement of the papillary muscles and the dilatation of the mitral valve annulus.

Dilation of the annulus of the mitral valve prevents the valve leaflets from fully coapting when the valve is closed. Mitral regurgitation of blood from the left ventricle into the left atrium results in increased total stroke volume and decreased cardiac output, and ultimate weakening of the left ventricle secondary to a volume overload and a pressure overload of the left atrium.

Chronic or acute left ventricular dilatation can lead to papillary muscle displacement with increased leaflet tethering due to tension on chordae tendineae, as well as annular dilatation.

US 7,431,692 to Zollinger et al. describes an adjustable support pad for adjustably holding a tensioning line used to apply tension to a body organ. The adjustable support pad can include a locking mechanism for preventing slidable movement of the tensioning element in one or both directions. The locking mechanism may include spring-loaded locks, rotatable cam-like structures, and/or rotatable spool structures. The adjustable support pad may be formed from rigid, semi-rigid, and/or flexible materials, and may be formed to conform to the outer surface of a body organ. The adjustable support pad can be configured to adjustably hold one or more separate tensioning lines, and to provide for independent adjustment of one or more tensioning lines or groups thereof.

US 2007/0118151 to Davidson describes a method and system to achieve leaflet coaptation in a cardiac valve percutaneously by creation of neochordae to prolapsing valve segments. This technique is especially useful in cases of ruptured chordae, but may be utilized in any segment of prolapsing leaflet. The technique described herein has the additional advantage of being adjustable in the beating heart. This allows tailoring of leaflet coaptation height under various loading conditions using image-guidance, such as echocardiography. This offers an additional distinct advantage over conventional open-surgery placement of artificial chordae. In traditional open surgical valve repair, chord length must be estimated in the arrested heart and may or may not be correct once the patient is weaned from cardiopulmonary bypass. The technique described below also allows for placement of multiple artificial chordae, as dictated by the patient's pathophysiology.

US 6,626,930 to Allen et al. describes apparatus and method for the stabilization and fastening of two pieces of tissue. A single device may be used to both stabilize and fasten the two pieces of tissue, or a separate stabilizing device may be used in conjunction with a fastening device. The stabilizing device may comprise a probe with vacuum ports and/or mechanical clamps disposed at the distal end to approximate the two pieces of tissue. After the pieces of tissue are stabilized, they are fastened together using sutures or clips. One exemplary application of a suture-based fastener comprises a toggle and suture arrangement deployed by a needle, wherein the needle enters the front side of the tissue and exits the blind side. In a second exemplary application, the suture-based fastener comprises a needle connected to a suture. The needle enters the blind side of the tissue and exits the front side. The suture is then tied in a knot to secure the pieces of tissue. One example of a clip-based fastener comprises a spring-loaded clip having two arms with tapered distal ends and barbs. The probe includes a deployment mechanism which causes the clip to pierce and lockingly secure the two pieces of tissue.

US 6,629,534 to St. Goar et al. describes methods, devices, and systems are provided for performing endovascular repair of atrioventricular and other cardiac valves in the heart. Regurgitation of an atrioventricular valve, particularly a mitral valve, can be repaired by modifying a tissue structure selected from the valve leaflets, the valve annulus, the valve chordae, and the papillary muscles. These structures may be modified by suturing, stapling, snaring, or shortening, using interventional tools which are introduced to a heart chamber. Preferably, the tissue structures will be temporarily modified prior to permanent modification. For example, opposed valve leaflets may be temporarily grasped and held into position prior to permanent attachment.

US 6,752,813 to Goldfarb et al. describes methods and devices for grasping, and optional repositioning and fixation of the valve leaflets to treat cardiac valve regurgitation, particularly mitral valve regurgitation. Such grasping will typically be atraumatic providing a number of benefits. For example, atraumatic grasping may allow repositioning of the devices relative to the leaflets and repositioning of the leaflets themselves without damage to the leaflets. However, in some cases it may be necessary or desired to include grasping which pierces or otherwise permanently affects the leaflets. In some of these cases, the grasping step includes fixation.

US 2003/0105519 to Fasol et al. describes artificial chordae having a strand member and a first and second pair of sutures at either longitudinal end of the strand member. The artificial chordae is preferably a unitary unit, formed from inelastic flexible material. In one application, the artificial chordae comprises multiple strand members joined together at a joined end. Different sized artificial chordae are provided sized to fit the patient's heart. The appropriately sized artificial chordae is chosen by using a chordae sizing gauge having a shaft and a transverse member, to measure the space within the patient's heart where the artificial chordae is attached.

US 2004/0010287 A1 to Bonutti describes an anchor having a pointed end portion that may be utilized to form an opening in a bone in a patient's body. The anchor is moved into the opening formed in the bone in the patient's body with a suture connected to the anchor. The suture may then be utilized to retain body tissue in a desired position relative to the bone. The body tissue may be either hard or soft body tissue. If desired, the anchor may be utilized in conjunction with layers of soft body tissue. When a suture is used it may be secured by connecting a retainer with the suture. Alternatively, sections of the suture may be interconnected. It is believed that it may be preferred to secure the suture in place after at least a predetermined tension has been established in the suture and/or a predetermined force has been transmitted to the body tissue. The suture may be secured in place by exposing a retainer to ultrasonic vibratory energy or by applying the ultrasonic vibratory energy directly to sections of the suture.

The following patents and patent application publications may be of interest:
PCT Publication WO 06/097931 to Gross et al.
PCT Publication WO 07/136783 to Cartledge et al.
PCT Publication WO 10/004546 to Gross et al.
PCT Publication WO 10/128502 to Maisano et al.
US 5,306,296 to Wright et al.
US 6,569,198 to Wilson et al.
US 6,619,291 to Hlavka et al.
US 6,764,510 to Vidlund et al.
US 7,004,176 to Lau
US 7,101,395 to Tremulis et al.
US 7,175,660 to Cartledge et al.
US 2003/0050693 to Quijano et al
US 2003/0167062 to Gambale et al.
US 2004/0024451 to Johnson et al.
US 2004/0148021 to Cartledge et al.
US 2004/0236419 to Milo
US 2005/0171601 to Cosgrove et al.
US 2005/0216039 to Lederman
US 2005/0288781 to Moaddeb et al.
US 2007/0016287 to Cartledge et al.
US 2007/0080188 to Spence et al.
US 2008/0262609 to Gross et al.
US 2009/0177266 to Powell et al.
US 2010/0161041 to Maisano et al.
US 2010/0161042 to Maisano et al.
US 2010/0161043 to Maisano et al.
US 2010/0280603 to Maisano et al.
US 2011/0106245 to Miller et al.

### SUMMARY OF THE INVENTION

According to the present invention, an apparatus as claimed in claim 1 is provided. Further embodiments are set forth in the dependent claims 2 to 15. The following Subject matter is not part of the present invention, but is useful for understanding the present invention:
Apparatus is provided comprising an implant comprising one or more primary adjustable repair chords and an adjustment mechanism that is configured to adjust a tension of the one or more adjustable repair chords and that is slidable along a guide wire toward an implantation site. Additionally, the apparatus may comprise a first tissue-engaging element (e.g., a tissue anchor) that may comprise one or more docking stations. Further additionally, a method is provided for implanting such apparatus. A respective guide wire may be reversibly coupled to each one of the docking stations. The adjustment mechanism may be slidable along the guide wire toward one of the one or more docking stations, and may be coupled to the tissue-engaging element via the docking station. Thus, the docking station may be a coupling element that provides coupling between two other elements (in this case, between adjustment mechanism and the tissue-engaging element.)

The repair chord may comprise a flexible, longitudinal member (e.g., sutures or wires). The repair chord may be coupled at a distal portion thereof to the adjustment mechanism. The repair cord may function as artificial chordae tendineae, or may be used to adjust a distance between two portions of the ventricular wall. The repair chord may be coupled at a proximal portion thereof to a second tissue-engaging element (e.g., a tissue anchor which penetrates or clips a portion of tissue).

Alternatively the repair chord comprises a cord that is disposed within at least a portion of an annuloplasty ring structure (e.g., a full annuloplasty ring or a partial annuloplasty ring). In this case, the annuloplasty ring structure comprises the adjustment mechanism that is coupled to the repair cord. The annuloplasty ring structure may be slidable along the guide wire toward one of the one or more docking stations, and may be coupled to the tissue-engaging element via the docking station. It is to be noted that the annuloplasty ring structure may be provided independently of the adjustment mechanism and the repair chord. In this case, the annuloplasty ring structure is slidable along the guide wire toward one of the one or more docking stations, and is coupled to the tissue-engaging element via the docking station.

A prosthetic heart valve and/or a support for the prosthetic heart valve may be slidable along the guide wire toward one of the one or more docking stations, and coupled to the tissue-engaging element via the docking station.

Thus, the tissue-engaging element and the docking station can be used to facilitate implantation of an implant such as cardiac valve implants, namely annuloplasty ring structures, prosthetic valves, and/or apparatus for receiving a prosthetic valve (e.g., a docking station or a support for receiving the prosthetic valve).

Typically, during a transcatheter procedure, the first tissue-engaging element is coupled to a first portion of tissue at a first implantation site in a heart of a patient. The adjustment mechanism is then slid along the guide wire and toward the first tissue-engaging element at the first implantation site. The proximal portion of the repair chord is then coupled via the second tissue-engaging element to a second portion of tissue at a second implantation site. Following the coupling of the second tissue-engaging element to the second implantation site, the adjustment mechanism is further slid distally toward the first tissue-engaging element and is then coupled to the first tissue-engaging element via the one or more docking stations on the first tissue-engaging element. Following the coupling of the adjustment mechanism to the second tissue-engaging element, a length and tension of the repair chord is then adjusted in order to adjust a distance between the first and second implantation sites. If the repair chord functions as an artificial chordae tendineae, the adjustment of the length and tension of the repair chord draws the leaflets together, and/or pulls the leaflet down toward the first implantation site to repair the valve.

The adjustment mechanism may comprise a spool assembly which adjusts a degree of tension of the repair chord. The spool assembly may comprise a housing, which houses a spool to which a distal portion of the repair chord is coupled.

If the repair chord is coupled to two respective portions of the ventricular wall, the two portions may be drawn together, thereby restoring the dimensions of the heart wall to physiological dimensions, and drawing the leaflets toward one another.

The adjustment mechanism may comprise a reversible locking mechanism which facilitates bidirectional rotation of the spool in order to effect both tensioning and relaxing of the repair chord. That is, the spool may be wound in one direction in order to tighten the repair chord, and in an opposite direction in order to slacken the repair chord. Thus, the spool adjustment mechanism may facilitate bidirectional adjustment of the repair chord.

The adjustable repair chord may be implanted during an open-heart or minimally-invasive procedure. In this case, the delivery tool comprises a handle and a multilumen shaft that is coupled at a distal end thereof to the adjustment mechanism. The delivery tool functions to advance the adjustment mechanism to the first portion of tissue, implant the adjustment mechanism at the first portion of tissue, and effect adjustment of the repair chord by effecting rotation of the spool. If the repair chord functions as an artificial chordae tendineae, prior to implantation of the adjustment mechanism, the distal portion of the delivery tool and the adjustment mechanism coupled thereto are advanced between the leaflets of the atrioventricular valve and into the ventricle toward the first portion of tissue. The incision made in the heart is then closed around the delivery tool and the heart resumes its normal function during the adjustment of the length of the artificial chordae tendineae.

Some embodiments of the apparatus and method described herein may be used for providing artificial chordae tendineae in a left ventricle of the heart and effecting adjustment thereof. Some embodiments of the apparatus and method described herein may be used for providing artificial chordae tendineae in a right ventricle of the heart and effecting adjustment thereof. Some embodiments of the apparatus and method described herein may be used for providing a system to adjust a length between two portions of the heart wall. Some embodiments of the apparatus and method described herein may be used for providing a docking station for an annuloplasty ring or for a prosthetic valve.

A method may comprise:
coupling a tissue-engaging element to a first portion of cardiac tissue of a heart of a patient;
advancing toward the tissue-engaging element an adjustment mechanism along at least a portion of at least one guide member that is removably coupled to the tissue-engaging element, the adjustment mechanism engaging at least a first portion of at least a first flexible longitudinal member;
coupling a second portion of the first flexible longitudinal member to a second portion of cardiac tissue;
following the coupling of the second portion of the first flexible longitudinal member to the second portion of cardiac tissue:
   sliding the adjustment mechanism further along the guide member; and
   coupling the adjustment mechanism to the tissue-engaging element; and
using the adjustment mechanism, adjusting a length of the first flexible longitudinal member between the first and second portions of cardiac tissue.

In the method, coupling the tissue-engaging element to the first portion of cardiac tissue may comprise coupling the tissue-engaging element to a papillary muscle of a ventricle of the patient.

In the method, coupling the tissue-engaging clement to the first portion of cardiac tissue may comprise coupling the tissue-engaging element to a portion of an inner wall of a ventricle of the patient.

In the method, adjusting the length of the flexible longitudinal member may comprise adjusting a distance between the first and second portions of cardiac tissue.

In the method, adjusting the length of the flexible longitudinal member may comprise adjusting the length of the flexible longitudinal member during beating of the heart of the patient.

In the method, adjusting the length of the flexible longitudinal member may comprise adjusting the length of the flexible longitudinal member during a first period thereof, and the method may further comprise further adjusting the length of the flexible longitudinal member during a second period that is after the first period.

In the method, coupling the tissue-engaging element to the first portion of cardiac tissue may comprise coupling the tissue-engaging element to an intracardiac portion of tissue in a manner in which a distal portion of the tissue-engaging element does not extend beyond an epicardium of the heart of the patient.

In the method, coupling the second portion of the flexible longitudinal member to the second portion of cardiac tissue may comprise coupling the second portion of the flexible longitudinal member to at least one leaflet of an atrioventricular valve of the patient.

In the method, coupling the second portion of the flexible longitudinal member to the second portion of cardiac tissue may comprise coupling the second portion of the flexible longitudinal member to exactly one leaflet of an atrioventricular valve of the patient.

In the method, coupling the second portion of the flexible longitudinal member to the second portion of cardiac tissue may comprise coupling, to a leaflet of an atrioventricular valve, a clip that is coupled to the second portion of the flexible longitudinal member.

In the method, coupling the adjustment mechanism to the tissue-engaging element may comprise locking the adjustment mechanism to a docking station coupled to the tissue-engaging element.

In the method, advancing the adjustment mechanism may comprise transcatheterally advancing the adjustment mechanism.

In the method, advancing the adjustment mechanism may comprise threading the guide member through an opening in the adjustment mechanism prior to the advancing.

The method may further comprise:
advancing a first portion of a second flexible longitudinal member, toward the tissue-engaging element; and
coupling a second portion of the second flexible longitudinal member to a third portion of cardiac tissue. Then, the method may further comprise coupling the first portion of the second flexible longitudinal member to the tissue-engaging element following the coupling of the second portion of the second flexible longitudinal member to the third portion of cardiac tissue. Alternatively, in the method:
   the second portion of cardiac tissue may include a portion of tissue of a ventricle of the patient,
   coupling the second portion of the first flexible longitudinal member to the second portion of cardiac tissue may comprise coupling the second portion of the first flexible longitudinal member to the portion of tissue of the ventricle of the patient,
   the third portion of cardiac tissue may include at least one leaflet of an atrioventricular valve of the heart of the patient, and
   coupling the second portion of the second flexible longitudinal member to the third portion of cardiac tissue may comprise coupling the second portion of the second flexible longitudinal member to the at least one leaflet of the atrioventricular valve.

In the method, coupling the tissue-engaging element to the first portion of tissue may comprise coupling a tissue-engaging element coupled to at least first and second docking stations, the first and second docking stations being removably coupled to first and second guide members, respectively. Then, in the method:
advancing the adjustment mechanism may comprise:
   advancing a first adjustment mechanism along the first guide member, the first adjustment mechanism engaging at least a first portion of at least a first flexible longitudinal member; and
   coupling the first adjustment mechanism to the first docking station, and
the method further may comprise:
   advancing a second adjustment mechanism along the second guide member, the second adjustment mechanism engaging at least a first portion of at least a second flexible longitudinal member; and
   coupling the second adjustment mechanism to the second docking station. Additionally, in the method, coupling the second portion of the flexible longitudinal member to the second portion of cardiac tissue may comprise coupling a second portion of the first flexible longitudinal member to the second portion of cardiac tissue, and the method may further comprise coupling a second portion of the second flexible longitudinal member to a third portion of cardiac tissue. Still additionally, in the method:
      the second portion of cardiac tissue may include a portion of tissue of a ventricle of the patient,
      coupling the second portion of the first flexible longitudinal member to the second portion of cardiac tissue may comprise coupling the second portion of the first flexible longitudinal member to the portion of tissue of the ventricle of the patient,
      the third portion of cardiac tissue may include at least one leaflet of an atrioventricular valve of the heart of the patient, and
      coupling the second portion of the second flexible longitudinal member to the third portion of cardiac tissue may comprise coupling the second portion of the second flexible longitudinal member to the at least one leaflet of the atrioventricular valve.

In the method, coupling the second portion of the flexible longitudinal member to the second portion of cardiac tissue may comprise coupling the second portion of the flexible longitudinal member to a portion of a wall of a ventricle of the patient, and adjusting the length of the flexible member may comprise adjusting a distance between the portion of the wall and the first portion of cardiac tissue.

In the method, adjusting the distance between the portion of the wall and the first portion of cardiac tissue may comprise adjusting a malpositioning of the heart wall of the patient.

In the method, the adjustment mechanism may include a spool coupled to the first portion of the flexible longitudinal member, and adjusting the length of the flexible longitudinal member using the adjustment mechanism may comprise rotating the spool.

Then, the method may further comprise unwinding a portion of the at least one flexible longitudinal member from around the spool, and adjusting the length of the flexible longitudinal member may comprise applying tension to the flexible longitudinal member subsequently to the unwinding. Alternatively, in the method, adjusting the length of the flexible longitudinal member may comprise:
applying tension to the flexible longitudinal member by winding successive portions of the flexible longitudinal member around the spool by rotating the spool in a first rotational direction thereof, and
slackening the flexible longitudinal member by unwinding the successive portions of the flexible longitudinal member from around the spool by rotating the spool in a second rotational direction thereof opposite the first rotational direction. Still alternatively, the method may further comprise unlocking the spool prior to the adjusting the length of the flexible longitudinal member, and locking the spool following the adjusting the length of the flexible longitudinal member.
In the method:
the second portion of tissue may include at least one leaflet of an atrioventricular valve of the patient,
the longitudinal member may comprise an artificial chordae tendineae, and
coupling the adjustment mechanism to the tissue-engaging element may comprise:
   advancing, between leaflets of the atrioventricular valve and into the ventricle, at least one shaft of a delivery tool, to which shaft the adjustment mechanism is removably coupled; and
   while the shaft remains coupled to the adjustment mechanism, coupling, using a coupling element holder of the delivery tool, at least one leaflet-engaging element to the at least one leaflet, wherein the second portion of the artificial chordae tendineae is coupled to the at least one leaflet-engaging element. Then, in the method, advancing the at least one shaft may comprise transcatheterally advancing the at least one shaft. Alternatively, in the method, coupling the at least one leaflet-engaging element to the at least one leaflet may comprise coupling the at least one leaflet-engaging element to exactly one leaflet.

Still alternatively, in the method, using the coupling element holder of the delivery tool may comprise sliding the coupling element holder with respect to the guide member.

In the method the at least one flexible longitudinal member may include first and second cord portions thereof, each of the first and second cord portions having respective free ends,
the first and second cord portions of the flexible longitudinal member may extend from the adjustment mechanism, and
coupling the second portion of the flexible longitudinal member to the second portion of cardiac tissue may comprise coupling each free end of the first and second cord portions to respective first and second leaflets of an atrioventricular valve of the patient. Then, in the method, adjusting the length of the flexible longitudinal member may comprise:
adjusting a length of the first cord portion of the longitudinal member between the adjustment mechanism and the first leaflet;
adjusting a length of the second cord portion of the longitudinal member between the adjustment mechanism and the second leaflet; and
by the adjusting the lengths of the first and second portions of the longitudinal member, drawing together the first and second leaflets.

Additionally, in the method, drawing together may comprise drawing together the first and second leaflets using a bead through which the first and second portions of the longitudinal member pass. Still additionally, in the method, using the bead may comprise advancing the bead to ventricular surfaces of the first and second leaflets, and advancing the bead to the ventricular surfaces may comprise creating an edge-to-edge repair of the first and second leaflets.

Another method may comprise:
coupling a guide member to a portion of tissue of a patient; and
advancing a tissue-adjustment mechanism toward the portion of tissue by:
   threading a portion of the guide member through at least one channel extending between a first opening in an upper surface of the tissue-adjustment mechanism and a second opening in a lower surface of the tissue-adjustment mechanism; and
   advancing the tissue-adjustment mechanism along the guide member and toward the portion of tissue.

The method may further comprise removing entirely the guide member from the patient following the advancing the tissue-adjustment mechanism along the guide member. Alternatively, the method may further comprise, prior to the coupling the guide member to the portion of tissue, reversibly coupling the guide member to a tissue anchor, and coupling the guide member to the portion of tissue of the patient may comprise implanting the tissue anchor in the portion of tissue of the patient.

The present invention will be more fully understood from the following detailed description of embodiments thereof, taken together with the drawings, in which:

### BRIEF DESCRIPTION OF THE DRAWINGS

Figs. 1-2 are schematic illustrations of apparatus comprising a tissue-engaging element comprising a docking station coupled to a guide wire, in accordance with some applications of the present invention;
Fig. 3 is a schematic illustration of advancement of an adjustment mechanism along the guide wire toward the docking station of Figs. 1 and 2, in accordance with some applications of the present invention;
Figs. 4-5 are schematic illustrations of engaging a leaflet with a leaflet engaging element, in accordance with some applications of the present invention;
Fig. 6 is a schematic illustration of coupling of the adjustment mechanism of Fig. 3 to the docking station, in accordance with some applications of the present invention;
Figs. 7-9 are schematic illustrations of adjusting by the adjustment mechanism a length of a repair chord coupled to the adjustment mechanism, in accordance with some applications of the present invention;
Fig. 10 is a schematic illustration of the adjustment mechanism and the repair chord, in accordance with some other applications of the present invention;
Figs. 11-15 are schematic illustrations of a plurality of docking stations and a plurality of adjustment mechanisms, in accordance with some applications of the present invention;
Fig. 16 is a schematic illustration of wall-to-wall adjustment using the docking station, adjustment mechanism, and repair chord, in accordance with some applications of the present invention;
Fig. 17 is a schematic illustration of wall-to-wall adjustment and leaflet adjustment using the plurality of docking stations, the plurality of adjustment mechanisms, and the plurality of repair chords, in accordance with some applications of the present invention;
Fig. 18 is a schematic illustration of wall-to-wall adjustment using the docking station, adjustment mechanism, and repair chord, in accordance with some other applications of the present invention;,
Figs. 19-20 are schematic illustrations of adjustment of a valve of a patient from a middle portion of the valve, in accordance with some applications of the present invention; and
Fig. 21 is a schematic illustration of the tissue-engaging element and the docking station of Figs. 1 and 2 being used to facilitate a cardiac valve implant, in accordance with some applications of the present invention.

### DETAILED DESCRIPTION OF EMBODIMENTS

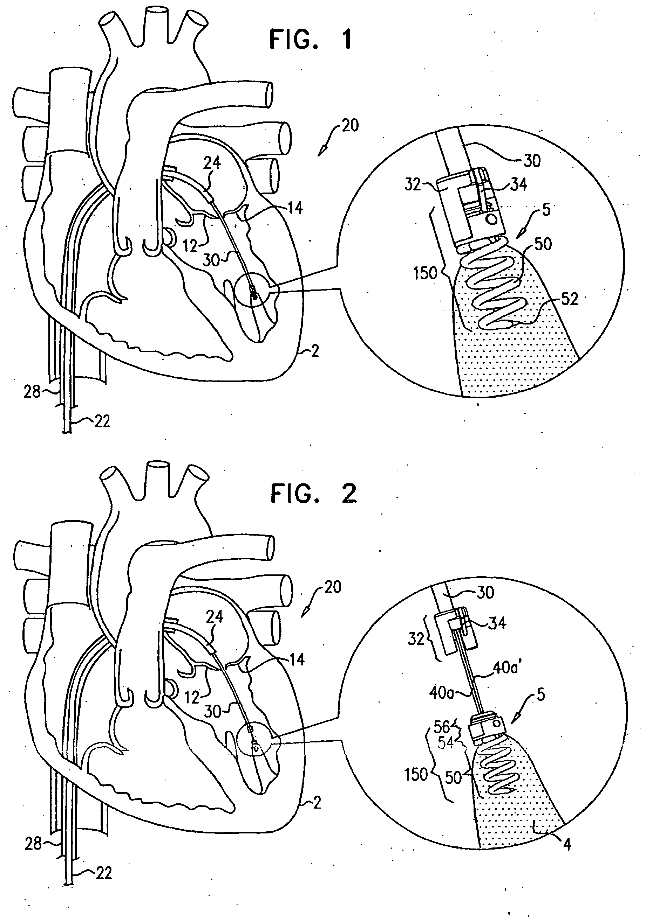

Reference is now made to Figs. 1-2, which are schematic illustrations of a system 20 comprising a docking assembly 150 for implantation at a first implantation site 5 of a patient, in accordance with some applications of the present invention. As shown in Fig. 2, docking assembly 150 comprises a tissue-engaging element having (1) a distal portion comprising a tissue anchor 50 (e.g., a helical tissue anchor as shown by way of illustration and not limitation), and (2) a proximal portion comprising a docking platform 54, and at least one docking station 56. Thus, docking assembly 150 comprises (a) the distal portion which engages the tissue of the patient (i.e., the tissue-engaging element), and (b) the proximal portion which is coupled to docking station 56. At least one guide member, (e.g., a guide wire 40, shown in Fig. 2) is reversibly coupled to docking assembly 150 (e.g., by being looped around, or otherwise coupled to, a portion of assembly 150) so as to define first and second portions 40a and 40a' that extend away from assembly 150.

Tissue anchor 50 is typically implanted within cardiac tissue in a manner in which a distal portion of anchor 50 does not extend beyond an epicardium of heart 2 of the patient. Thus, anchor 50 is implanted at an intracardiac site such that the implant, (e.g., the adjustment mechanism or an implant comprising the adjustment mechanism) that is eventually coupled thereto (as described hereinbelow) is implanted at the intracardiac site such that no portions of the adjustment mechanism extend beyond the epicardium of the heart.

Docking assembly 150 and guide wire 40 are advanced toward implantation site typically during a transcatheter procedure, as shown. However, it is to be noted that the scope of the present invention includes the advancement of assembly 150 and guide wire 40 during a minimally-invasive or open-heart procedure. The procedure is typically performed with the aid of imaging, such as fluoroscopy, transesophageal echo, and/or echocardiography.

The transcatheter procedure typically begins with the advancing of a semi-rigid guide wire into a right atrium of the patient. The semi-rigid guide wire provides a guide for the subsequent advancement of a sheath 28 therealong and into the right atrium. Once sheath 28 has entered the right atrium, the semi-rigid guide wire is retracted from the patient's body. Sheath 28 typically comprises a 13-20 F sheath, although the size may be selected as appropriate for a given patient. Sheath 28 is advanced through vasculature into the right atrium using a suitable point of origin typically determined for a given patient. For example:
- sheath 28 may be introduced into the femoral vein of the patient, through an inferior vena cava, into the right atrium, and into the left atrium transseptally, typically through the fossa ovalis;
- sheath 28 may be introduced into the basilic vein, through the subclavian vein to the superior vena cava, into the right atrium, and into the left atrium transseptally, typically through the fossa ovalis; or
- sheath 28 may be introduced into the external jugular vein, through the subclavian vein to the superior vena cava, into the right atrium, and into the left atrium transseptally, typically through the fossa ovalis.

In some applications of the present invention, sheath 28 is advanced through the inferior vena cava of the patient (as shown) and into the right atrium using a suitable point of origin typically determined for a given patient.

Sheath 28 is advanced distally until the sheath reaches the interatrial septum. For some applications, a resilient needle and a dilator (not shown) are advanced through sheath 28 and into the heart. In order to advance sheath 28 transseptally into the left atrium, the dilator is advanced to the septum, and the needle is pushed from within the dilator and is allowed to puncture the septum to create an opening that facilitates passage of the dilator and subsequently sheath 28 therethrough and into the left atrium. The dilator is passed through the hole in the septum created by the needle. Typically, the dilator is shaped to define a hollow shaft for passage along the needle, and the hollow shaft is shaped to define a tapered distal end. This tapered distal end is first advanced through the hole created by the needle. The hole is enlarged when the gradually increasing diameter of the distal end of the dilator is pushed through the hole in the septum.

The advancement of sheath 28 through the septum and into the left atrium is followed by the extraction of the dilator and the needle from within sheath 28. Subsequently, a docking-assembly delivery tool 30 is advanced through sheath 28. Tool 30 is typically advanced within a lumen of an advancement sheath 22 having a distal end 24. Advancement sheath 22 is advanced within sheath 28. Delivery tool 30 is coupled at a distal end thereof to a manipulator 32 which is reversibly coupled to docking station 56 and docking platform 54 of docking assembly 150. Manipulator 32 has (1) lateral arms which cup platform 54, and (2) a docking-station-coupler 34, as shown in Fig. 1. Coupler 34 is biased to move radially-inward, as shown in Fig. 1. Docking station 56 is ribbed, such that coupler 34, when moved radially inward, engages at least one rib of docking station 56, thereby coupling assembly 150 to delivery tool 30.

Delivery tool 30 and manipulator 32 are shaped so as to define a lumen for passage therethrough of guide wire 40.

Docking assembly 150 is implanted in implantation site 5 by rotating tool 30 in order to rotate anchor 50 and corkscrew anchor 50 into tissue of site 5. Site 5 typically comprises a portion of tissue at an intraventricular site in heart 2 of the patient. As shown, site 5 includes a papillary muscle 4, by way of illustration and not limitation. It is to be noted that site 5 includes any portion of cardiac tissue, e.g., a portion of a free wall of the ventricle, a portion of the septum facing the ventricle, a portion of tissue at a base of the papillary muscle, or a portion of the wall at the apex of the ventricle. (For the purposes of the claims, "a portion of tissue of a ventricle" includes any portion of cardiac tissue, e.g., a portion of a free wall of the ventricle, a portion of the septum facing the ventricle, a portion of tissue at a base of the papillary muscle, or a portion of the wall at the apex of the ventricle.)

Following the implantation of assembly 150 at site 5, tool 30 is disengaged from assembly 150 when the physician pulls on tool 30. This pulling pulls on manipulator 32 such that coupler 34 is actively moved radially outward against the ribs of docking station 56, and is thereby decoupled from station 56. At the time of pulling, tissue at implantation site 5 pulls on assembly 150 (in the direction opposite the direction of pulling by the physician) so as to help disengage tool 30 from assembly 150.

As shown in Fig. 2, following the decoupling of tool 30 from assembly 150, tool 30 is pulled proximally along guide wire 40 and is extracted from the body of the patient together with advancement sheath 22, leaving behind assembly 150 and guide wire 40.

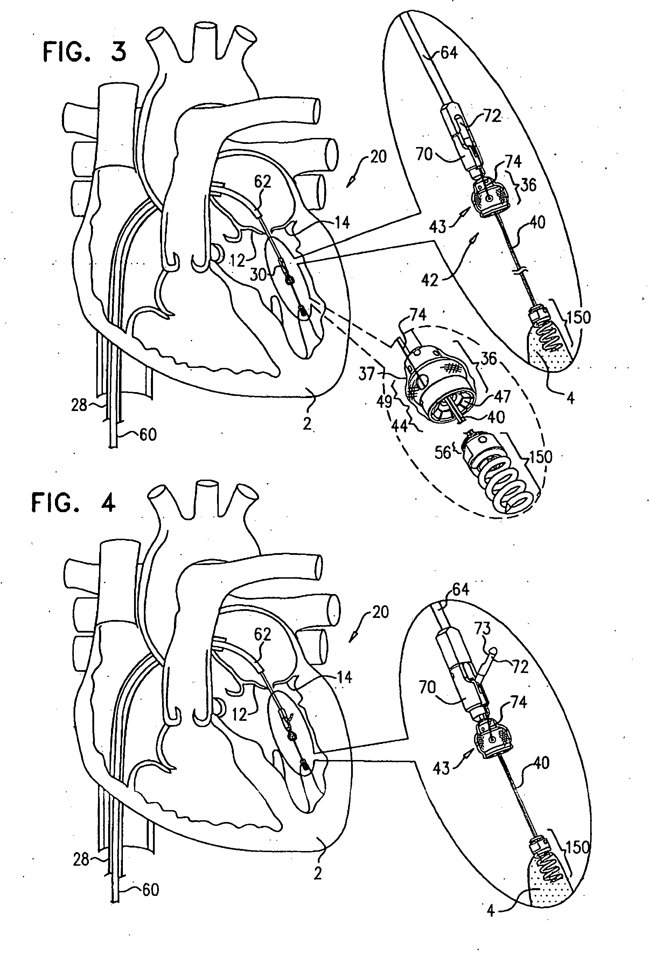

Fig. 3 shows advancement of an implant (e.g., a spool assembly 36 comprising an adjustment mechanism 43) along guide wire 40 by an adjustment-mechanism delivery tool 64, in accordance with some applications of the present invention. Tool 64 is surrounded by and slidable within an advancement sheath 60 having a distal end 62.

Spool assembly 36 is surrounded by a braided fabric mesh, e.g., a polyester mesh, which promotes fibrosis around assembly 36 and facilitates coupling of assembly 36 to tissue of heart 2. Assembly 36 houses a rotatable structure (e.g., a spool as shown hereinbelow) that is surrounded by a housing 49. Housing 49 is coupled to a distal cap 44 which facilitates coupling of assembly 36 to docking station 56 of docking assembly 150. As shown, cap 44 is shaped so as to define a plurality of baffles 47 that are disposed angularly with respect to a distal end of cap 44. Baffles 47 are coupled to the distal end of cap 44 along respective coupling joints which facilitate movement of each baffle 47. During the coupling of spool assembly 36 to docking station 56, the ribbed portion of docking station 56 pushes inwardly baffles 47 of cap 44, as is described hereinbelow. Baffles 47 then expand and engage an area of docking station 56 between the ribs of the ribbed portion so as to dock and lock assembly 36 to docking station 56.

Additionally, cap 44 is shaped so as to define a central opening therethrough which facilitates passage therethrough of guide wire 40. Additionally, spool assembly 36 and the components thereof are shaped so as to define a central opening (i.e., an opening having the same axis as guide wire 40). That is, spool 46 has a central opening, and housing 49 has a central opening which facilitates passage of spool 46 and housing 49 along guide wire 40.

As shown, adjustment mechanism 43 is coupled to a distal portion of a repair chord 74 (e.g., repair chord 74 is looped through or otherwise coupled to a portion of adjustment mechanism 43). Chord 74 comprises a flexible longitudinal member. For some applications, and as is described hereinbelow, chord 74 functions as an artificial chordea tendinea. A proximal portion of chord 74 is coupled to a leaflet-engaging element 72 (e.g., a clip, as shown). Leaflet-engaging element 72 is disposed within a holder 70 that is coupled to delivery tool 64. Chord 74 a superelastic, biocompatible material (e.g., nitinol, ePTFE, PTFE, polyester, stainless steel, or cobalt chrome). Typically, chord 74 comprises an artificial chordea tendinea.

Figs. 4-5 are schematic illustrations of the engaging of leaflet-engaging element 72 to at least one leaflet 14 of a mitral valve of the patient, in accordance with some applications of the present invention. As shown in Fig. 4, the clip is opened from a remote location outside the body of the patient.

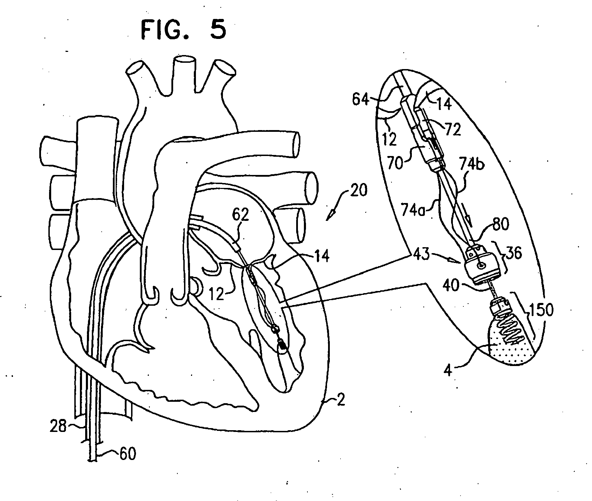

For some applications, the clip typically is shaped so as to define at least one coupling protrusion 73. The clip has a tendency to close, and is initially held open by a cord (not shown) that is coupled to a surface of the clip, extends through delivery tool 64, and is held taught outside of the heart. Once the clip has been advanced to the desired location on the leaflet, the cord is relaxed, allowing the clip to close. The cord is removed, typically by releasing one end thereof and pulling the other end. The positioning of holder 70 between the leaflets (Fig. 5) helps ensure that the clip engages exactly one of the leaflets. It is noted that in Fig. 5 the clip is shown engaging only a single leaflet (leaflet 14). The clip typically engages the leaflet by clamping the leaflet such that the clip engages atrial and ventricular surfaces of the leaflet. The clip may puncture the leaflet, or may merely press firmly against the leaflet.

It is to be noted that the scope of the present invention include the clipping together of both leaflets 12 and 14. For applications in which system 20 is used to repair a tricuspid valve of the patient, the clip may clip any one, two, or all three leaflets together.

Holder 70 is shaped to define a groove which houses the clip during the advancement of tool 64 toward the ventricle. The groove functions as a track to facilitate slidable detachment of the clip from holder 70 following the engaging of the clip to leaflet 14.

Alternatively, the clip has a tendency to open. In order to close the clip, a cord is provided. A distal-most portion of the cord is looped around the clip. Once the clip has been advanced to the desired location on the leaflet, as shown in Fig. 5, the surgeon pulls on both ends of the cord, thereby causing the clip to become locked closed. The cord is removed, typically by releasing one end thereof and pulling the other end.

It is to be noted that the scope of the present invention includes any leaflet-engaging element known in the art.

As shown in Fig. 5, portions 74a and 74b extend from leaflet-engaging element 72 toward adjustment mechanism 43. Portions 74a and 74b define portions of a single chord 74 that is looped through a portion of mechanism 43. Alternatively, portions 74a and 74b represent two distinct chords which are coupled at their distal ends to adjustment mechanism 43 and at their proximal ends to leaflet-engaging element 72.

As shown, leaflet-engaging element 72 engages leaflet 14 prior to coupling spool assembly 36 to docking station 56.

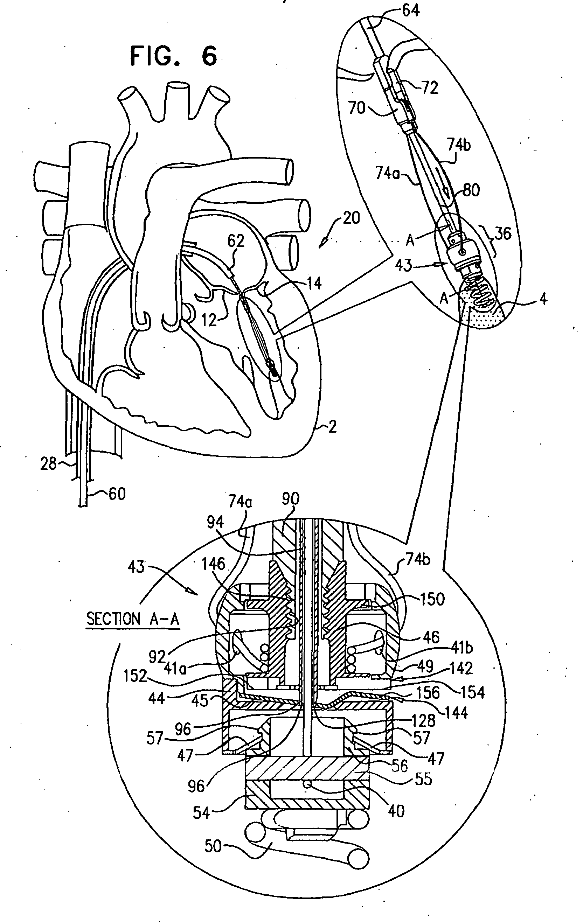

Fig. 6 shows spool assembly 36 being coupled to docking station 56, in accordance with some applications of the present invention. Following the coupling of leaflet-engaging element 72 to leaflet 14, spool assembly 36 is pushed distally toward docking station 56. Spool assembly 36 is coupled to an advancement shaft 80 which pushes assembly 36. Shaft 80 slides within a lumen of delivery tool 64 and within a lumen of holder 70 so as to advance spool assembly 36, while leaflet-engaging element 72 remains engaged with leaflet 14. Advancement shaft 80 functions to advance distally spool assembly 36 and functions to facilitate engagement between spool assembly 36 and docking station 56.

As described hereinabove, docking station 56 has one or more locking mechanisms (e.g., one or more ribs 57, shown in the enlarged cross-sectional image of Fig. 6) which project laterally such that rib 57 defines a shelf and an depressed area underneath the shelf (i.e., the cross-sectional diameter at rib 57 is larger than the cross-sectional diameter at the area underneath the shelf). As described hereinabove, cap 44 of assembly 36 is shaped so as to define a plurality of baffles 47. As cap 44 engages docking station 56, baffles 47 are pushed inward and upward angularly as each baffle slides against rib 57. After each baffle 47 passes the shelf of rib 57, the baffle engages the depressed area underneath the shelf of rib 57, as shown in the enlarged cross-sectional image of Fig. 6. The shelf of rib 57 prevents upward movement of baffles 47 and thereby locks in place baffles 47 and cap 44 with respect to docking station 56. Rib 57, therefore, comprises a locking mechanism so as to lock implant 42 (e.g., adjustment mechanism 43) to tissue anchor 50.

Following the coupling of assembly 36 to docking station 56, spool 46 is rotated in a first rotational direction in order to advance with respect to spool 46 and contact with spool 46 successive portions of chord 74. For example, when the successive portions of chord 74 are advanced with respect to spool 46, the successive portions of chord 74 are looped around spool 46. The rotating of spool 46 in the first rotational direction pulls tight and adjusts a length of chord 74 between leaflet 14 and spool 46, in order to adjust a distance between leaflet 14 and implantation site 5 and to facilitate coaptation between leaflets 12 and 14, as is described hereinbelow.

Housing 49 is shaped so as to provide openings 41a and 41b for passage therethrough of portions 74a and 74b, respectively, of chord 74 into housing 49. For some applications of the present invention, portions 74a and 74b define portions of a single chord 74 that is looped through spool 46. For other applications, portions 74a and 74b define discrete chords which are each coupled at respective distal ends thereof to spool 46.

The enlarged, cross-sectional image of Fig. 6 shows spool 46 within housing 49. Spool 46 defines an upper surface 150, a lower surface 152, and a cylindrical body portion disposed vertically between surfaces 150 and 152. Spool 46 is shaped to provide a driving interface, e.g., a channel, which extends from an opening provided by upper surface 150 to an opening provided by lower surface 152. A proximal portion of the driving interface is shaped to define a threaded portion 146 which may or may not be tapered. Threaded portion 146 of spool 46 is engageable by a threaded portion of a screwdriver head 92 of a screwdriver 90. Screwdriver 90 is coupled to a distal end of shaft 80. For some applications, shaft 80 rotates screwdriver 90. For other applications, shaft 80 is shaped so as to define a lumen for advancement therethrough of a screwdriver-rotation tool that facilitates rotation of screwdriver 90. Rotation of screwdriver 90 and screwdriver head 92 rotates spool 46, as the respective threaded portions of spool 46 and screwdriver head 92 engage. The cylindrical body portion of spool 46 is shaped to define one or more holes which function as respective coupling sites for coupling (e.g., looping through the one or more holes, or welding to spool 46 in the vicinity of the one or more holes) of any number of chords 74 to spool 46.

Lower surface 152 of spool 46 is shaped to define one or more (e.g., a plurality, as shown) recesses 154 which define structural barrier portions 155 of lower surface 152. It is to be noted that any suitable number of recesses 154 may be provided, e.g., between 1 and 10 recesses, circumferentially or otherwise, with respect to lower surface 152 of spool 46.

As shown, a locking mechanism 45 is disposed in communication with lower surface 152 of spool 46 and disposed in communication with at least in part to a lower surface of housing 49. Typically, a cap 44 maintains locking mechanism 45 in place with respect to lower surface 152 of spool 46 and lower surface of housing 49. For some applications, locking mechanism 45 is coupled, e.g., welded, to the lower surface of housing 49. Typically, locking mechanism 45 defines a mechanical element having a planar surface that defines slits. It is to be noted that the surface of locking mechanism 45 may also be curved, and not planar. Locking mechanism 45 is shaped to provide a protrusion 156 which projects out of a plane defined by the planar surface of the mechanical element. The slits of mechanism 45 define a depressible portion 128 that is disposed in communication with and extends toward protrusion 156. Depressible portion 128 is moveable in response to a force applied thereto typically by an elongate locking mechanism release rod 94 which slides through a lumen of screwdriver 90 and a torque-delivering tool that is coupled thereto.

It is to be noted that the planar, mechanical element of locking mechanism 45 is shown by way of illustration and not limitation and that any suitable mechanical element having or lacking a planar surface but shaped to define at least one protrusion may be used together with locking mechanism 45.

Cap 44 is provided that is shaped to define a planar surface and an annular wall having an upper surface thereof. The upper surface of the annular wall is coupled to, e.g., welded to, a lower surface provided by housing 49. The annular wall of cap 44 is shaped to define a recessed portion 144 of cap, 44 that is in alignment with a recessed portion 142 of spool housing 49.

As shown, a distal end 96 of locking mechanism release rod 94 pushes distally on depressible portion 128 in order to unlock locking mechanism 45 from spool 46. Pushing depressible portion 128 by locking mechanism release rod 94 pushes distally protrusion 156 within recessed portion 142 of housing 49 and within recessed portion 144 of cap 44, which frees protrusion 156 from recesses 154 of spool 46. Once protrusion 156 is released from recesses 154 of spool 46, the physician is able to rotate spool 46 bidirectionally in order to adjust a tension of chord 74.

When the physician rotates spool 46 in the first rotational direction, chord 74 is pulled tight, and leaflet 14 is drawn toward adjustment mechanism 40 and toward anterior leaflet 12 of mitral valve 8.

In the resting state (i.e., prior to the rotation of spool 46 in order to adjust chord 74, following coupling of leaflet-engaging element 72 to leaflet 14) chord 74 is wrapped around spool 46 a few times (e.g., three times, by way of illustration and not limitation). This winding provides excess slack to chord 74 (in case portions 74a and 74b are coupled too tightly to leaflet 14). If the physician wishes to provide slack to member 74 or to any one of portion 74a or 74b, the physician unwinds a bit of the wrapped portion of member 74 from around spool 46 (e.g., by unwinding chord 74 a few times from around spool 46, or by unwinding chord 74 entirely from around spool 46 so that chord 74 slides freely through spool 46 within a channel provided therein). In order to accomplish such unwinding, the physician rotates spool 46 in a rotational direction in which it unwinds the wrapped portion of chord 74. Since chord 74 is looped through spool 46 in the channel provided therein, when chord 74 is unwound from spool 46, the physician can pull on one or both portions 74a and 74b so as to adjust, make even, or further slacken any one of or both portions 74a and 74b that extend from spool 46.

When the physician desires to pull tight chord 74, he or she effects rotation of spool 46 in a first rotational direction, i.e., the direction opposite the second rotational direction in which spool 46 is rotated during the unwinding of chord 74 from spool 46. Rotation of spool 46 in the first rotational direction winds chord 74 around spool 46, while rotation of spool 46 in a second rotational direction that is opposite the first rotational direction, unwinds the portion of longitudinal chord 74 from around spool 46.

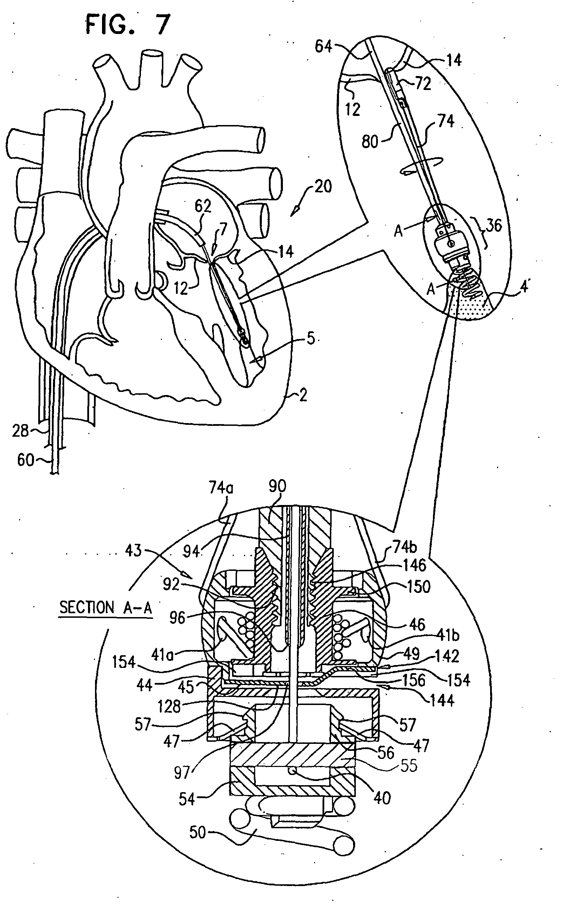

Fig. 7 shows spool assembly 36 following the adjustment of chord 74 by rotating screwdriver 90 in the direction as indicated by the arrow, and the partial removal of screwdriver 90, in accordance with some applications of the present invention. As shown in the enlarged cross-sectional image of Fig. 7, successive portions of chord 74 are wrapped around spool 46. That is, chord 74 is wrapped more times around spool 46 following adjustment (e.g., an additional 4 times, as shown in Fig. 7), than prior to adjustment (Fig. 6). This pulls chord 74 from a slackened state (Fig. 6) to a taut state (Fig. 7) in order to adjust a length of chord 74 between adjustment mechanism 43 and the proximal end of chord 74 that is coupled to leaflet-engaging element 72. Additionally, this applying of tension to chord 74 adjusts a length between first and second implantation sites 5 and 7. Typically, chord 74 is adjusted while heart 2 is beating.

As shown, rod 94 is shaped so as to define a central lumen and a distal opening for passage therethrough of guide wire 40. Additionally, depressible portion 128 is shaped so as to provide an opening for passage of guide wire 40 therethrough. Guide wire 40 is looped around a distal looping element 55 of docking platform 54 of docking assembly 150. Following the adjusting of the tension and length of chord 74, screwdriver 90 is decoupled from spool 46 (e.g., by being unscrewed from threaded portion 146 of spool 46) and is advanced proximally together with rod 94 away from spool assembly 36, as shown in the enlarged, cross-sectional image of Fig. 7.

Following the decoupling of screwdriver 90 from spool 46 and the removal of screwdriver 90, guide wire 40 remains coupled to docking platform 54 and docking assembly 150. Guide wire 40 then facilitates subsequent advancement of screwdriver 90 or any other tool to access spool assembly 36 and/or to facilitate further adjustment of chord 74 beyond the initial adjustment. Guide wire 40 may remain chronically coupled to docking assembly 150 and may be accessible at a subcutaneous location of the patient, e.g., a port. For other applications, guide wire 40 is removed from docking assembly 150 when the physician determines that further adjustment of chord 74 is not needed. The physician removes guide wire 40 by pulling, from outside the body of the patient, one end of guide wire 40 so that guide wire 40 slides around element 55 and is unlooped therefrom. The physician continues to pull on the end of guide wire 40 until the second end of wire 40 is exposed and removed from the patient.

Following the removal of locking-mechanism release rod 94, depressible portion 128 is no longer depressed by distal end 96 of rod 94, and protrusion 156 returns within a recess 154 of spool 46 so as to lock spool 46 in place and restriction rotation thereof in either direction (Fig. 7).

Reference is now made to Figs. 3-7. It is to be noted that spool assembly 36 is only coupled to docking assembly 150 following the coupling of leaflet-engaging element 72 to leaflet 14. This is done in order to reduce the strain on implantation site 5. Should spool assembly 36 be implanted at implantation site 5 prior to engaging leaflet 14 with leaflet-engaging element 72, more strain would be applied to implantation site 5 than if spool assembly 36 had been implanted following the coupling of leaflet-engaging element 72 to leaflet 14, as described herein. That is, the pulling force is applied in a downward direction from leaflet 14 toward implantation site 5 instead of from implantation site 5 upward toward leaflet 14.

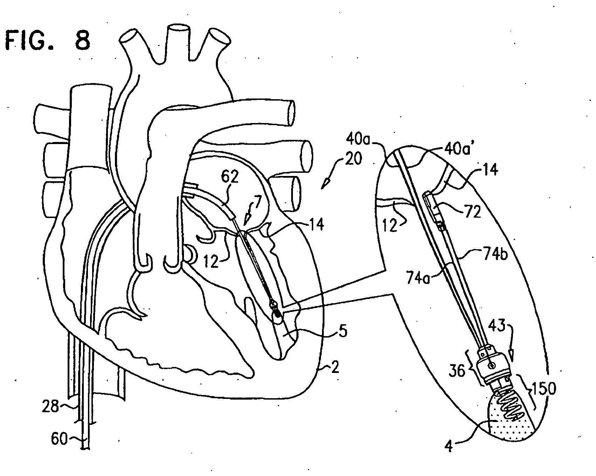

Fig. 8 shows system 20 following the removal of the tool used to rotate spool 46 of spool assembly 36, in accordance with some applications of the present invention. As shown, chord 74 is pulled tight such that its length and tension are adjusted, and leaflet 14 is pulled and adjusted commensurate with the adjustment of chord 74. Guide wire 40 remains coupled to spool assembly 36 and to docking assembly 150, as shown, such that portions 40a and 40a' extend from spool assembly 36. Guide wire 40 facilitates the reintroduction of the tool used to rotate spool 46, or of any other tool.

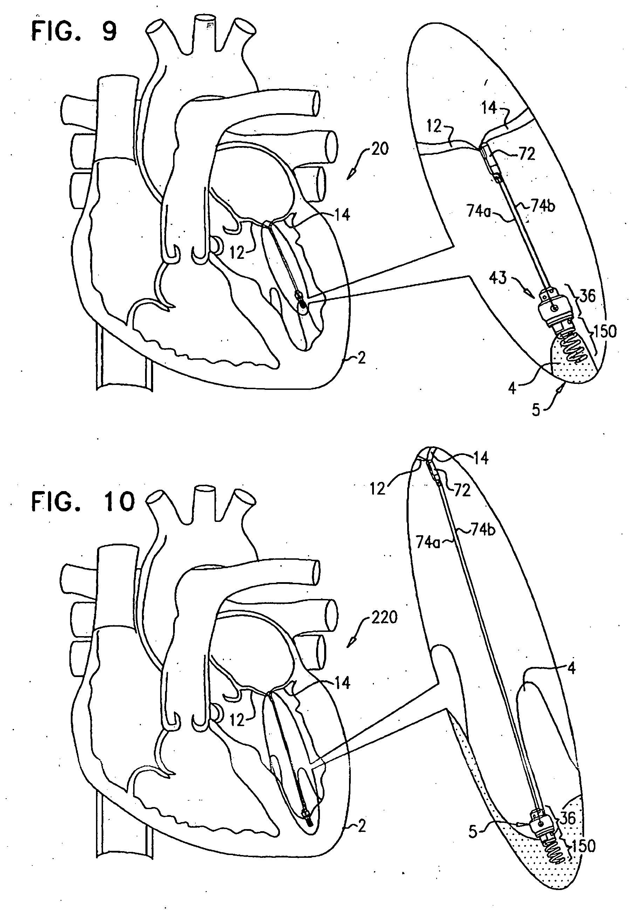

Fig. 9 shows system 20 following the removal of guide wire 40 from heart 2, in accordance with some applications of the present invention. As shown, the adjustment of chord 74 draws leaflets 12 and 14 together. It is to be noted that although leaflet-engaging element 72 is shown as engaging only leaflet 14, the scope of the present invention includes the engaging of both leaflets 12 and 14 by leaflet-engaging element 72.

Fig. 10 shows a system 220, as described hereinabove with reference to system 20, with the exception that implantation site 5 includes tissue of the wall of the ventricle at the base of papillary muscle 4 in a vicinity of the apex of the heart, in accordance with some applications of the present invention. Implantation site 5 is shown by way of illustration and not limitation, and as described hereinabove, site 5 may include any portion of tissue of heart 2. It is to be noted that although leaflet-engaging element 72 is shown as engaging only leaflet 14, the scope of the present invention includes the engaging of both leaflets 12 and 14 by leaflet-engaging element 72.

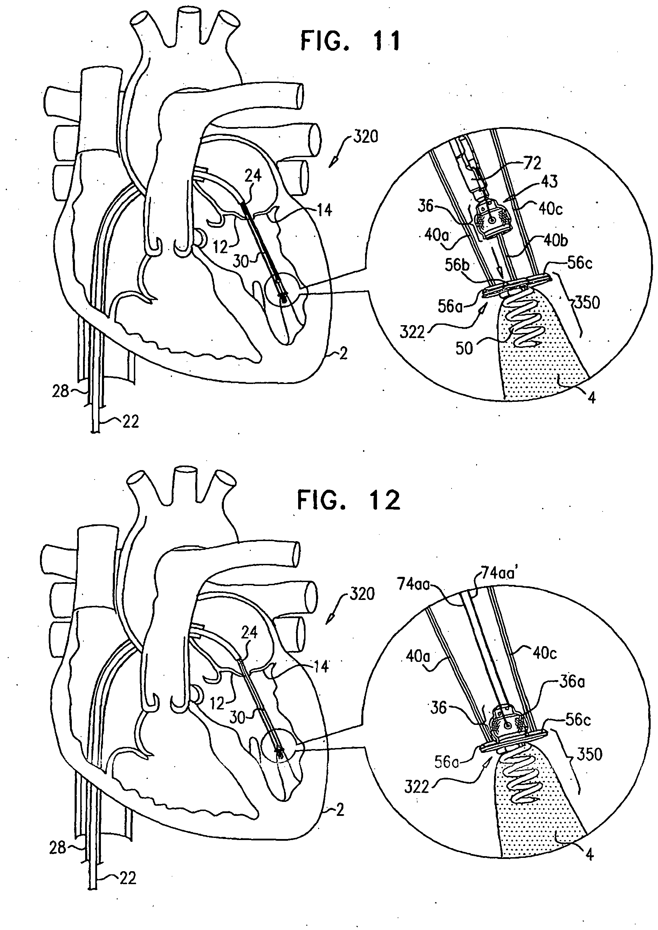

Figs. 11-15 are schematic illustrations of a system 320 comprising a multiple-docking-station assembly 350 comprising a plurality of docking stations 56, in accordance with some applications of the present invention. Multiple-docking-station assembly 350 comprises a tissue anchor 50 and a docking platform 322 which supports two or more docking stations 56. Platform 322, as shown, supports three docking stations 56a, 56b, and 56c, by way of illustration and not limitation. It is to be noted that platform 322 may support any number of docking stations 56. As shown, each docking station 56a, 56b, and 56c is reversibly coupled to a respective guide wire 40a, 40b, and 40c, in a manner as described hereinabove. Each docking station 56a, 56b, and 56c facilitates coupling thereto of a respective spool assembly 36a, 36b, and 36c, or any other tool or device which may be coupled to docking stations 56a, 56b, and 56c.

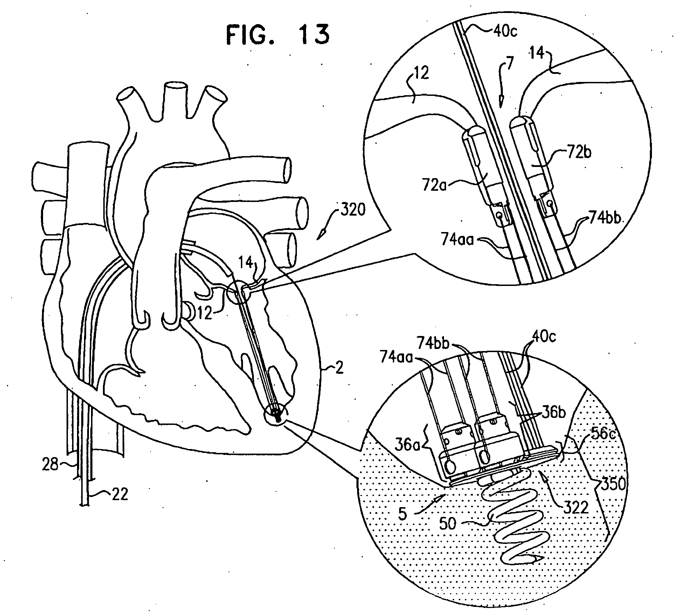

As shown in Figs. 11-13, first and second spool assemblies 36a and 36b are coupled via respective guide wires 40a and 40b to respective docking stations 56a and 56b. Each spool assembly 36a and 36b has a respective chord 74aa and 74bb extending therefrom (Fig. 13). For example (as shown in Fig. 12), the chord extending from spool assembly 36a has portions 74aa and 74aa' extending from spool assembly 36a. Each chord 74 is coupled to a respective leaflet-engaging element 72. That is, chord 74aa is coupled to leaflet-engaging element 72a, and chord 74bb is coupled to leaflet-engaging element 72b (Fig. 13).

Each leaflet-engaging element 72a and 72b is coupled to leaflets 12 and 14, respectively, and then each spool assembly 36a and 36b is coupled to respective docking stations 56a and 56b, in a manner as described hereinabove. Chords 74aa and 74bb are then adjusted, as described hereinabove. Each chord 74aa and 74bb may be adjusted sequentially or simultaneously.

Fig. 13 shows chords 74aa and 74bb following their adjustment. The relative dispositions of leaflets 12 and 14 are adjusted in conjunction with the adjusting of chords 74aa and 74bb. Typically, leaflets 12 and 14 are drawn together to repair the heart valve.

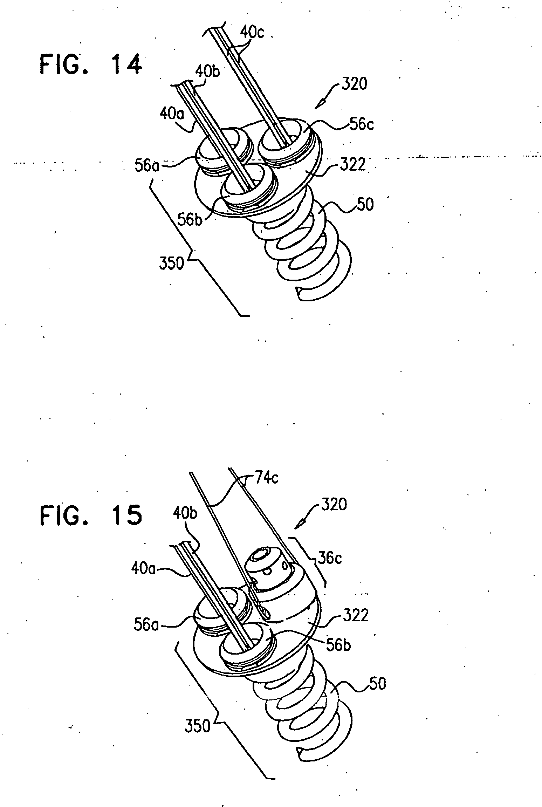

As shown in Fig. 15, a third spool assembly 36c may be coupled to docking station 56c. Chord 74c coupled thereto may be coupled to a third implantation site in heart 2 and subsequently adjusted. Fig. 15 shows third spool assembly 36c coupled to docking station 56c without the presence of the other spool assemblies 36a and 36b, by way of illustration and not limitation.

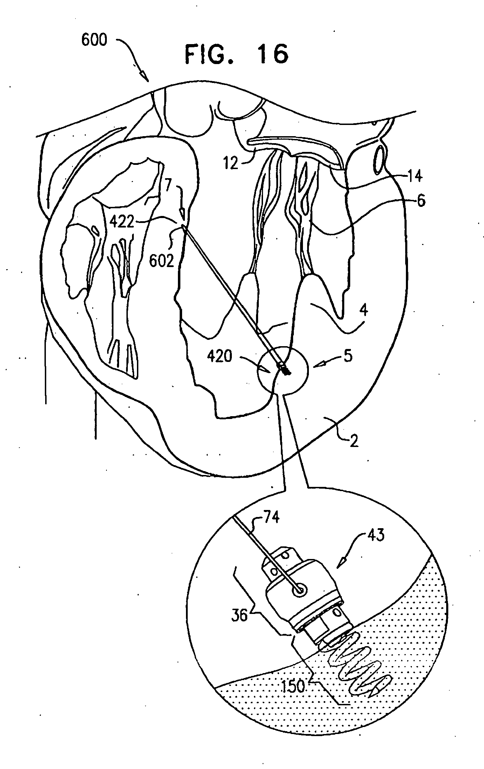

Fig. 16 shows a system 600 for repairing malpositioning of the wall of the ventricle of the patient, in accordance with respective applications of the present invention. System 600 treats a weakened state of heart 2 in which the wall of the left ventricle is malpositioned and weakened. As a result of the malpositioning of the wall of the heart, leaflets 12 and 14 of mitral valve 8 are malpositioned and are distanced from one another (not shown). In order to treat the malpositioning of the heart wall and thereby of leaflets 12 and 14, spool assembly 36 is implanted at a first portion 420 of heart tissue which faces and surrounds the left ventricle of heart 2. First implantation site 5 thus comprises first portion 420 of heart tissue. It is to be noted that first implantation site 5 is at the base of the papillary muscle by way of illustration and not limitation, and that first implantation site 5 may be at a portion of the wall of the heart in a vicinity of the apex of the heart, or at papillary muscle 4. For some applications in which system 600 treats malpositioning of the heart, docking assembly 350 and spool assembly 36 are implanted externally to the ventricle, and chord 74 extends through cardiac tissue and into the ventricle toward implantation site 7.

Spool assembly 36 is implanted via docking assembly 150 at site 5 in a manner as described hereinabove with reference to Figs. 3-6. As shown, the free ends of chord 74 are coupled to a second portion 422 of heart tissue which faces and surrounds the left ventricle of heart 2. Second implantation site 7 thus comprises second portion 422 of heart tissue, e.g., at the septum, by way of illustration and not limitation. The free ends of longitudinal chord 74 are coupled to the heart tissue using any suitable attachment means 602, e.g., sutures, knotting, or tissue anchors such as helical anchors. Spool 46 of adjustment mechanism 43 is rotated, as described hereinabove, thereby pulling tight chord 74 and thereby reducing a length of chord 74 between first and second implantation sites 5 and 7. In response to the pulling of chord 74, first and second portions 420 and 422 of the heart tissue are pulled toward one another, and a length of chord 74 is adjusted. Consequently, the dimensions of the heart wall are restored to physiological dimensions, and leaflets 12 and 14 are drawn toward one another.

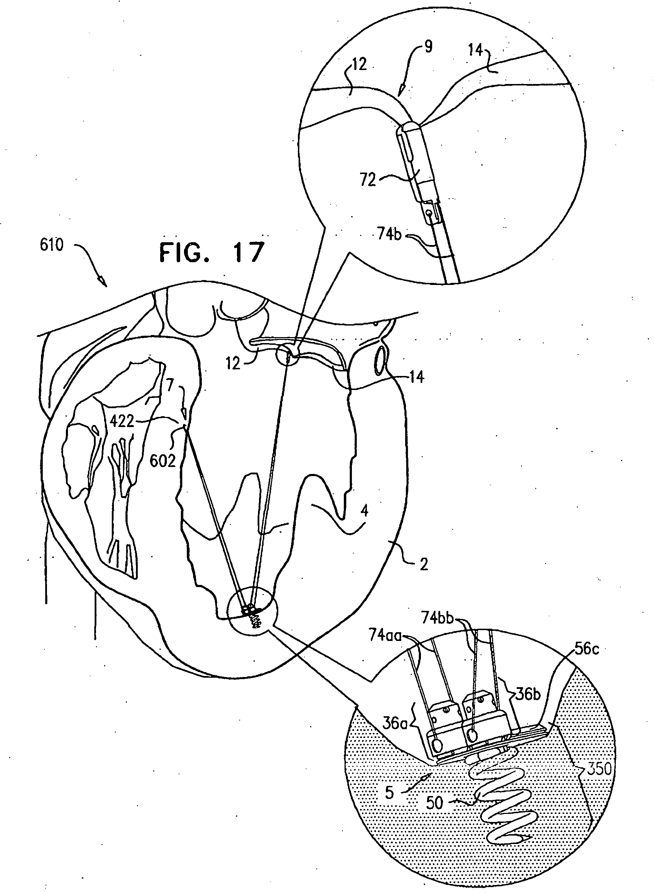

Fig. 17 shows a system 610 for adjusting both malpositioning of a heart wall of heart 2, and a relative disposition of leaflet 12, in accordance with some applications of the present invention. Multiple-docking-station assembly 350 is implanted at implantation site 5, i.e., a portion of tissue of a heart wall of heart 2 in a vicinity of the apex of heart 2. It is to be noted that implantation site 5 may include any portion of tissue of heart 2, e.g., a portion of tissue at the base of papillary muscle 4, a portion of tissue of papillary muscle 4, or a portion of the free wall of the ventricle. As described hereinabove, first spool assembly 36a is coupled to docking station 56a and adjusts a length of chord 74aa in order to adjust a distance between implantation sites 5 and 7. Second spool assembly 36b is coupled to docking station 56b and adjusts a length of chord 74bb in order to adjust a distance between implantation site 5 a third implantation site 9 (e.g., leaflet 12, as shown). As described hereinabove, chords 74aa and 74bb may be adjusted simultaneously or sequentially. Following the adjusting, implantation sites 7 and 9 are drawn toward multiple-docking-station assembly 350 at implantation site 5. Consequently, the dimensions of the heart wall are restored to physiological dimensions, and leaflets 12 and 14 are drawn toward one another. It is to be noted that although leaflet-engaging element 72 is shown as engaging only leaflet 12, the scope of the present invention includes the engaging of both leaflets 12 and 14 by leaflet-engaging element 72.

It is to be further noted that the scope of the present invention includes the coupling of a third spool assembly to docking station 56c coupled to chord 74c. For such applications, the free end of chord 74c may be coupled to a different portion of cardiac tissue, e.g., leaflet 14.

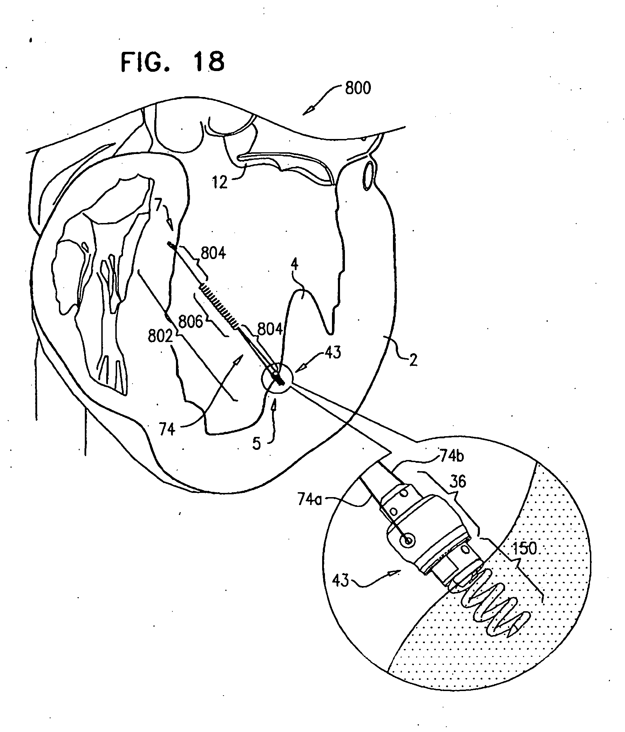

Fig. 18 is a schematic illustration of a system 800 for adjusting a distance between two portions of a heart wall of the left ventricle of the patient, in accordance with some applications of the present invention. System 800 comprises a tensioning device 802 coupled at a first end thereof to spool assembly 36 at docking assembly 150. In a manner as described hereinabove, spool assembly 36 is implanted at first implantation site 5 in a first portion of tissue of the heart wall that faces and surrounds the ventricular lumen. The free end of tensioning device 802 is attached at second implantation site 7 to a second portion of tissue of the heart wall that faces and surrounds the ventricular lumen. The free end of tensioning device 802 is implanted in heart tissue using a helical anchor by way of illustration and not limitation. For example, the free end of tensioning device 802 may be coupled to second implantation site 7 using sutures, knots, or any tissue anchor known in the art.

Tensioning device 802 comprises a flexible material, e.g., ePTFE or nitinol, and is shaped to define a coiled portion 806 that has a length of between 20 mm and 50 mm and a diameter of between 0.5 mm and 3.0 mm. Tensioning device 802 comprises respective wire/suture portions 804 on either side of coiled portion 806. For such an application, the suture portion 804 that is between spool assembly 36 and coiled portion 806 comprises portions 74a and 74b of chord 74.

As described hereinabove, spool 46 of adjustment mechanism 43 is rotated in order to adjust a distance between first and second implantation sites 5 and 7. As spool 46 is rotated in a first direction thereof, successive portions of chord 74 of suture portion 804 that is disposed adjacently to spool assembly 36 are wrapped around spool 46. Tensioning device 802 is tightened and shortened in response to the wrapping of portion 804 around spool 46. As device 802 is tightened, a force is applied to coiled portion 806 of tensioning device 802. Coiled portion 806 applies a supplemental puling force to help pull the opposing first and second portions of the ventricle wall toward one another. Consequently, the dimensions of the heart wall are restored to physiological dimensions, and leaflets 12 and 14 are drawn toward one another.

Reference is made to Figs. 16-18. It is to be noted that the scope of the present invention includes the use of systems 600, 610, and 800 for adjusting a distance between any two portions of the heart and not just opposing portions, as described hereinabove. For example, first and second implantation sites 5 and 7 may be on the same side, e.g., the septum, of the wall of the heart.

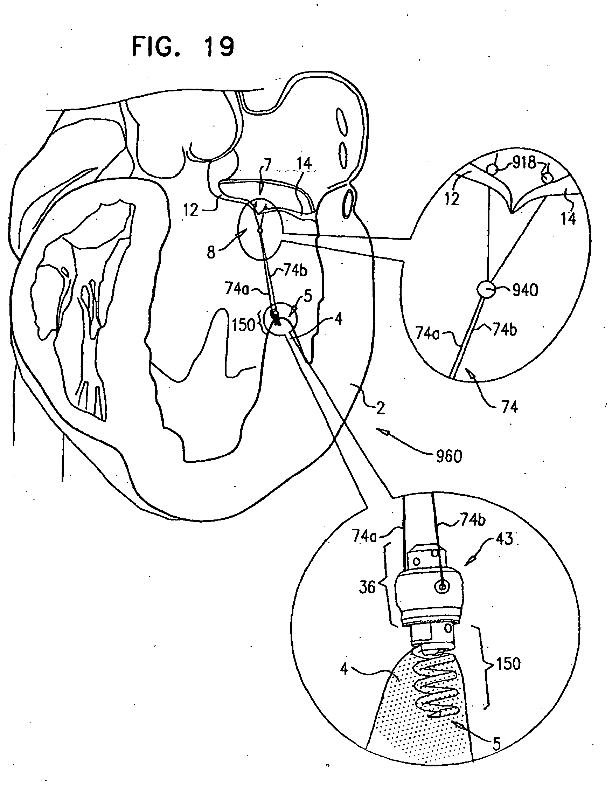

Reference is now made to Fig. 19, which is a schematic illustration of a system 960 for drawing together leaflets 12 and 14 of mitral valve 8 of the patient, in accordance with some applications of the present invention. Spool assembly 36 is implanted via docking assembly 150 in first implantation site 5 at papillary muscle 4 of the left ventricle by way of illustration and not limitation. For example, spool assembly 36 may be implanted in a portion of the heart wall of the ventricle, e.g., the base of the papillary muscle. First and second portions 74a and 74b of chord 74 are coupled (e.g., sutured, anchored, clipped, or locked in place with a crimping bead 918, as shown) to leaflet 12 at an implantation site 902. It is to be noted that portions 74a and 74b may be coupled to leaflets 12 and 14, respectively, using leaflet-engaging elements 72 as described hereinabove.

As described hereinabove, spool 46 of adjustment mechanism 43 is rotated in order to adjust a length of portions 74a and 74b of chord 74. Portions 74a and 74b are pulled tight in response to rotation of spool 46 in a first direction thereof. In response to the pulling of portions 74a and 74b, leaflets 12 and 14 are pulled toward one another in order to restore coaptation to valve 8.

It is to be noted that system 960 may be used on the tricuspid valve.

System 960 further comprises at least one bead 940 that is threaded over portions 74a and 74b of chord 74. The surgeon adjusts the position of the bead along the portions 74a and 74b in order to set the degree to which portions 74a and 74b are free to move with respect to one another. In general, as bead 940 is positioned closer to valve 8, portions 74a and 74b are more constrained in their motion with respect to one another, and leaflets 12 and 14 are drawn closer together. For some applications of the present invention, bead 940 comprises a fixation mechanism (e.g., a crimping mechanism), which is configured to fix the bead to portions 74a and 74b of chord 74 once bead 940 has been positioned at a desire location along portions 74a and 74b.

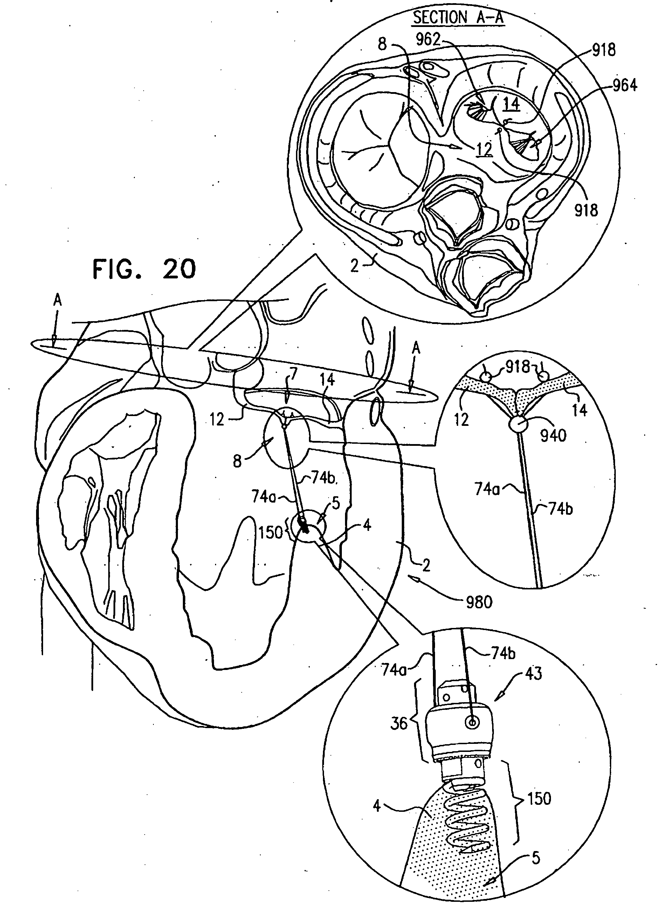

Fig. 20 shows a system 980 that is similar to system 960 as described with reference to Fig. 19, with the exception that bead 940 is pulled by the operating physician to the ventricular surface of a middle portion of valve 8, in accordance with some applications of the present invention. Such pulling of bead 940 to the ventricular surface creates a bridge between leaflets 12 and 14, e.g., as an Alfieri stitch, or edge-to-edge repair. Portions 74a and 74b are then adjusted in order to pull together the middle portion of mitral valve 8, as shown in Section A-A. The firm coupling of leaflets 12 and 14 prevents prolapsing of leaflets 12 and 14, facilitates coaptation of leaflets 12 and 14, and creates orifices 962 and 964 (section A-A) in mitral valve 8 so as to facilitate blood flow from the atrium to the ventricle. Additionally, the adjusting of portions 74a and 74b of chord 74 draws downward leaflets 12 and 14 and adjusts chord 74 such that it functions as an artificial chordea tendinea.

Reference is now made to Figs. 19 and 20. It is to be noted that although docking assembly 150 is shown, multiple-docking-station assembly 350 as described hereinabove, may be implanted at implantation site 5. For such an application, two or more spool assemblies 36 may be coupled to multiple-docking-station assembly 350, and any number of chords 74 extending from each spool assembly 36 may be coupled to leaflets 12 and 14 at any suitable location thereof. The lengths of chords 74 are then adjusted by spool assemblies 36 in order to pull leaflets 12 and 14 together.

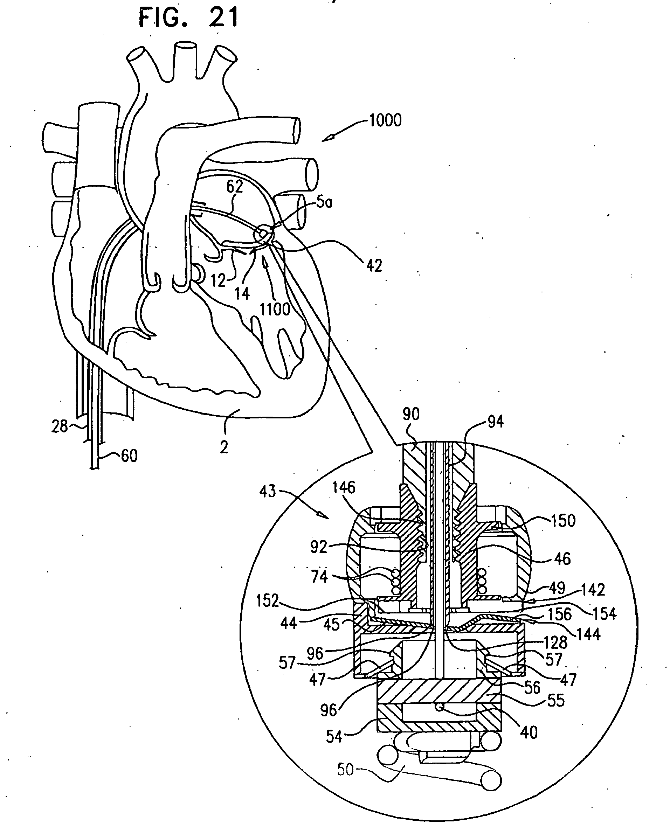

Reference is now made to Fig. 21, which is a schematic illustration of a system 1000 comprising docking assembly 150 for implantation at an implantation site 5a that includes an annulus 1100 of a cardiac valve of the patient, in accordance with some applications of the present invention. It is to be noted that the mitral valve is shown by way of illustration and not limitation, and that system 1000 can be used on any other cardiac valve of the patient, e.g., the tricuspid valve, the pulmonary valve, and the aortic valve. System 1000 comprises docking assembly 150 and the guide member coupled thereto (e.g., guide wire 40), as described hereinabove with reference to Figs. 1-2.

For some applications in which docking assembly 150 is implanted at the annulus of the cardiac valve, implant 42 configured to be coupled to docking assembly 150 comprises an annuloplasty ring structure (e.g., a full annuloplasty ring or a partial annuloplasty ring). Typically, the annuloplasty ring structure comprises adjustment mechanism 43. It is to be noted, however, that the annuloplasty ring structure configured to be coupled to docking assembly 150 may be provided independently of adjustment mechanism 43. That is, any suitable annuloplasty ring structure may be coupled to docking assembly 150. For such applications, the annuloplasty ring structure is slid along guide wire 40 toward docking assembly 150.

For other applications in which docking assembly 150 is implanted at the annulus of the cardiac valve, implant 42 configured to be coupled to docking assembly 150 comprises a prosthetic valve or a support structure for coupling a prosthetic valve thereto. For some applications, the support structure comprises adjustment mechanism 43. It is to be noted, however, that the support structure configured to be coupled to docking assembly 150 may be provided independently of adjustment mechanism 43. That is, any suitable support structure or prosthetic valve may be coupled to docking assembly 150. For such applications, the support structure or prosthetic valve is slid along guide wire 40 toward docking assembly 150.

For some applications of the present invention, systems 20, 220, 320, 600, 610, 800, 960, 980, and 1000 are used to treat an atrioventricular valve other than the mitral valve, i.e., the tricuspid valve. For these applications, systems 20, 220, 320, 600, 610, 800, 960, 980, and 1000 described hereinabove as being placed in the left ventricle are instead placed in the right ventricle.

It is to be noted that the scope of the present invention includes the use of systems 20, 220, 320, 600, 610, 800, 960, 980, and 1000 on other cardiac valves, such as the pulmonary valve or the aortic valve.

It is to be further noted that the scope of the present invention includes the use of systems 20, 220, 320, 600, 610, 800, 960, 980, and 1000 on other tissue other than cardiac tissue, e.g., gastric tissue or any other suitable tissue or organ.

For some applications, techniques described herein are practiced in combination with techniques described in one or more of the references cited in the Background section of the present patent application.

• In an application, techniques and apparatus described in one or more of the following applications are combined with techniques and apparatus described herein:
- PCT Publication WO 06/097931 to Gross et al., entitled, "Mitral Valve treatment techniques," filed March 15, 2006;
- US Provisional Patent Application 60/873,075 to Gross et al., entitled, "Mitral valve closure techniques," filed December 5, 2006;
- US Provisional Patent Application 60/902,146 to Gross et al., entitled, "Mitral valve closure techniques," filed on February 16, 2007;
- US Provisional Patent Application 61/001,013 to Gross et al., entitled, "Segmented ring placement," filed October 29, 2007;
- PCT Patent Application PCT/IL07/001503 to Gross et al., entitled, "Segmented ring placement," filed on December 5, 2007;
- US Patent Application 11/950,930 to Gross et al., entitled, "Segmented ring placement," filed on December 5, 2007, which published as US Patent Application Publication 2008/0262609;
- US Provisional Patent Application 61/132,295 to Gross et al., entitled, "Annuloplasty devices and methods of delivery therefor," filed on June 16, 2008;
- US Patent Application 12/341,960 to Cabiri, entitled, "Adjustable partial annuloplasty ring and mechanism therefor," filed on December 22, 2008, which published as 2010/0161047;
- US Provisional Patent Application 61/207,908 to Miller et al., entitled, "Actively-engageable movement-restriction mechanism for use with an annuloplasty structure," filed on February 17, 2009;
- US Patent Application 12/435,291 to Maisano et al., entitled, "Adjustable repair chords and spool mechanism therefor," filed on May 4, 2009, which published as 2010/0161041;
- US Patent Application 12/437,103 to Zipory et al., entitled, "Annuloplasty ring with intra-ring anchoring," filed on May 7, 2009, which published as 2010/0286767;
- PCT Patent Application PCT/IL2009/000593 to Gross et al., entitled, "Annuloplasty devices and methods of delivery therefor," filed on June 15, 2009, which published as WO 10/004546;
- US Patent Application 12/548,991 to Maisano et al., entitled, "Implantation of repair chords in the heart," filed on August 27, 2009, which published as 2010/0161042;
- US Patent Application 12/608,316 to Miller et al., entitled, "Tissue anchor for annuloplasty ring," filed on October 29, 2009, which published as 2011/0106247;
- PCT Patent Application PCT/IL2009/001209 to Cabiri et al., entitled, "Adjustable annuloplasty devices and mechanisms therefor," filed on December 22, 2009, which published as WO 10/073246;
- US Patent Application 12/689,635 to Zipory et al., entitled, "Over-wire rotation tool," filed on January 19, 2010, which published as 2010/0280604;
- US Patent Application 12/689,693 to Hammer et al., entitled, "Application Deployment techniques for annuloplasty ring," filed on January 19, 2010, which published as 2010/0280605;
- US Patent Application 12/706,868 to Miller et al., entitled, "Actively-engageable movement-restriction mechanism for use with an annuloplasty structure," filed on February 17, 2010, which published as 2010/0211166; and/or
- US Patent Application 12/795,026 to Miller et al., entitled, "Apparatus for guide-wire based advancement of a rotation assembly," filed on June 7, 2010, which published as 2011/0106245.

## Claims

1. Apparatus for use with at least one implant (42), comprising:
a tissue-engaging element having (a) a distal portion configured to engage at least a first portion of tissue of a patient, and (b) a proximal portion;
at least one docking station (56) coupled to the proximal portion of the tissue-engaging element, the at least one docking station (56):
being configured to receive and be coupled to the at least one implant (42), and
comprising a locking mechanism (57) configured to lock the implant (42) to the docking station (56); and
at least one guide member (40) reversibly coupled to the at least one docking station (56), the at least one guide member (40) being configured for facilitating slidable advancement of the at least one implant (42) toward the docking station (56).

2. The apparatus according to claim 1, wherein the at least one docking station (56) comprises two or more docking stations, and wherein the at least one guide member (40) comprises two or more guide members, each guide member (40) being reversibly coupled to a respective docking station (56).

3. The apparatus according to claim 1, wherein the implant (42) comprises at least one implant selected from the group consisting of: a prosthetic cardiac valve and a support for receiving a prosthetic cardiac valve, and wherein the at least one docking station (56) is configured to receive and be coupled to the selected implant (42).

4. The apparatus according to claim 1, wherein the implant (42) comprises a tissue-adjustment device selected from the group consisting of: a partial annuloplasty ring and a full annuloplasty ring, and wherein the at least one docking station (56) is configured to receive and be coupled to the selected tissue-adjustment device.

5. The apparatus according to any one of claims 1-4, further comprising the implant (42).

6. The apparatus according to claim 5, wherein the implant (42) has:
an upper surface (150) and a lower surface (152),
at least one first opening at the upper surface (150),
at least one second opening at the lower surface (152), and
a channel extending between the first and second openings, the channel facilitating advancement of the implant along the guide member (40).

7. The apparatus according to claim 6, wherein the implant (42) comprises a first coupling, and wherein the locking mechanism (57) comprises a second coupling configured to be coupled to the first coupling.

8. The apparatus according to claim 7, wherein the second coupling (57) comprises at least one depressed portion, and wherein the first coupling comprises at least one moveable baffle (47) which is configured to engage the at least one depressed portion of the second coupling.

9. The apparatus according to any one of claims 1-2, further comprising at least one flexible longitudinal member (74) coupled at a first portion thereof to the implant (42), wherein a second portion of the flexible longitudinal member (74) is configured to be coupled to a second portion of tissue of the patient, and wherein the implant (42) is configured to adjust a length of the longitudinal member (74) between the first and second portions of tissue.

10. The apparatus according to claim 9, wherein:
the first portion of tissue includes a first portion of cardiac tissue at a first intraventricular site (5),
the second portion of tissue includes at least one leaflet (12, 14) of an atrioventricular valve of the patient, and
the flexible longitudinal member (74) comprises at least one artificial chordea tendinea.

11. The apparatus according to claim 9, wherein:
the implant (42) comprises a rotatable structure (46),
the at least one flexible longitudinal member (74) is coupled at the first portion to the rotatable structure (46), and
the rotatable structure (46) is bidirectionally rotatable to adjust the degree of tension of the at least one flexible longitudinal member (74).

12. The apparatus according to claim 11, wherein the rotatable structure (46) is configured such that:
rotation of the rotatable structure (46) in a first rotational direction applies tension to the flexible longitudinal member (74), and
rotation of the rotatable structure (46) in a second rotational direction that is opposite the first rotational direction slackens the flexible longitudinal member (74).

13. The apparatus according to claim 11, further comprising a rotatable structure locking mechanism (45) displaceable with respect to the rotatable structure (46), so as to:
release the rotatable structure (46) during rotation of the rotatable structure - (46), and
lock in place the rotatable structure (46) following rotation of the rotatable structure (46).

14. The apparatus according to claim 11, wherein the rotatable structure (46) comprises a spool (46), and wherein the at least one flexible longitudinal member (74) is configured to be wound around the spool (46) during the rotation of the spool (46) in a first rotational direction.

15. The apparatus according to any one of claims 1-4, wherein:
the implant (42) comprises a rotatable structure (46), coupled to a flexible longitudinal member (74),
the rotatable structure (46) is bidirectionally rotatable to adjust a degree of tension of the flexible longitudinal member (74), and
the at least one docking station (56) is configured to receive and be coupled to the rotatable structure (46).

## Patentansprüche

1. Vorrichtung zur Verwendung mit zumindest einem Implantat (42), umfassend:
ein Gewebeeingriffselement mit (a) einem distalen Abschnitt, der dafür ausgelegt ist, mit zumindest einem ersten Gewebeabschnitt eines Patienten in Eingriff zu treten, und (b) einem proximalen Abschnitt;
zumindest eine Anschlussstation (56), die mit dem proximalen Abschnitt des Gewebeeingriffselements gekoppelt ist, wobei die zumindest eine Anschlussstation (56):
dafür ausgelegt ist, das zumindest eine Implantat (42) aufzunehmen und mit diesem gekoppelt zu werden, und
einen Arretiermechanismus (57) umfasst, der dafür ausgelegt ist, das Implantat (42) an der Anschlussstation (56) zu arretieren; und
zumindest ein Führungselement (40), das reversibel mit der zumindest einen Anschlussstation (56) gekoppelt ist, wobei das zumindest eine Führungselement (40) dafür ausgelegt ist, ein gleitendes Vorschieben des zumindest einen Implantats (42) zu der Anschlussstation (56) hin zu erleichtern.

2. Vorrichtung nach Anspruch 1, wobei die zumindest eine Anschlussstation (56) zwei oder mehr Anschlussstationen umfasst und wobei das zumindest eine Führungselement (40) zwei oder mehr Führungselemente umfasst, wobei ein jedes Führungselement (40) reversibel mit einer entsprechenden Anschlussstation (56) gekoppelt ist.

3. Vorrichtung nach Anspruch 1, wobei das Implantat (42) zumindest ein Implantat umfasst, das aus der Gruppe bestehend aus: einer prothetischen Herzklappe und einem Träger zur Aufnahme einer prothetischen Herzklappe ausgewählt ist, und wobei die zumindest eine Anschlussstation (56) dafür ausgelegt ist, das ausgewählte Implantat (42) aufzunehmen und mit diesem gekoppelt zu werden.

4. Vorrichtung nach Anspruch 1, wobei das Implantat (42) zumindest eine Gewebeeinstellvorrichtung umfasst, die aus der Gruppe bestehend aus: einem partiellen Anuloplastiering und einem vollen Anuloplastiering ausgewählt ist, und wobei die zumindest eine Anschlussstation (56) dafür ausgelegt ist, die ausgewählte Gewebeeinstellvorrichtung aufzunehmen und mit dieser gekoppelt zu werden.

5. Vorrichtung nach einem der Ansprüche 1-4, ferner umfassend das Implantat (42).

6. Vorrichtung nach Anspruch 5, wobei das Implantat (42) aufweist:
eine Oberseite (150) und eine Unterseite (152),
zumindest eine erste Öffnung an der Oberseite (150),
zumindest eine zweite Öffnung an der Unterseite (152), und
einen Kanal, der sich zwischen der ersten und der zweiten Öffnung erstreckt, wobei der Kanal ein Vorschieben des Implantats entlang des Führungselements (40) erleichtert.

7. Vorrichtung nach Anspruch 6, wobei das Implantat (42) ein erstes Koppelteil aufweist und wobei der Arretiermechanismus (57) ein zweites Koppelteil aufweist, das dafür ausgelegt ist, mit dem ersten Koppelteil gekoppelt zu werden.

8. Vorrichtung nach Anspruch 7, wobei das zweite Koppelteil (57) zumindest einen vertieften Abschnitt umfasst und wobei das erste Koppelteil zumindest eine bewegliche Ablenkplatte (47) umfasst, die dafür ausgelegt ist, mit dem zumindest einen vertieften Abschnitt des zweiten Koppelteils in Eingriff zu treten.

9. Vorrichtung nach einem der Ansprüche 1-2, ferner umfassend zumindest ein flexibles Längselement (74), das an einem ersten Abschnitt davon mit dem Implantat (42) gekoppelt ist, wobei ein zweiter Abschnitt des flexiblen Längselements (74) dafür ausgelegt ist, mit einem zweiten Gewebeabschnitt des Patienten gekoppelt zu werden, und wobei das Implantat (42) dafür ausgelegt ist, eine Länge des Längselements (74) zwischen dem ersten und dem zweiten Gewebeabschnitt einzustellen.

10. Vorrichtung nach Anspruch 9, wobei:
der erste Gewebeabschnitt einen ersten Herzgewebeabschnitt an einem ersten intraventrikulären Ort (5) umfasst,
der zweite Gewebeabschnitt zumindest ein Segel (12, 14) einer Atrioventrikularklappe des Patienten umfasst, und
das flexible Längselement (74) zumindest einen künstlichen Sehnenfaden (chordae tendineae) umfasst.

11. Vorrichtung nach Anspruch 9, wobei:
das Implantat (42) eine drehbare Struktur (46) umfasst,
das zumindest eine flexible Längselement (74) an dem ersten Abschnitt mit der drehbaren Struktur (46) gekoppelt ist, und
die drehbare Struktur (46) bidirektional drehbar ist, um den Spannungsgrad des zumindest einen flexiblen Längselements (74) einzustellen.

12. Vorrichtung nach Anspruch 11, wobei die drehbare Struktur (46) derart ausgelegt ist, dass:
durch eine Drehung der drehbaren Struktur (46) in einer ersten Drehrichtung eine Spannung auf das flexible Längselement (74) ausgeübt wird, und
durch eine Drehung der drehbaren Struktur (46) in einer zweiten Drehrichtung, welche der ersten Drehrichtung entgegengesetzt ist, die auf das flexible Längselement (74) ausgeübte Spannung gelockert wird.

13. Vorrichtung nach Anspruch 11, ferner umfassend einen Arretiermechanismus (45) der drehbaren Struktur, welcher in Bezug auf die drehbare Struktur (46) derart verschiebbar ist, dass er:
die drehbare Struktur (46) während des Drehens der drehbaren Struktur (46) freigibt, und
die drehbare Struktur (46) nach dem Drehen der drehbaren Struktur (46) an Ort und Stelle arretiert.

14. Vorrichtung nach Anspruch 11, wobei die drehbare Struktur (46) eine Spule (46) umfasst und wobei das zumindest eine flexible Längselement (74) dafür ausgelegt ist, während des Drehens der Spule (46) in einer ersten Drehrichtung um die Spule (46) herumgewickelt zu werden.

15. Vorrichtung nach einem der Ansprüche 1-4, wobei:
das Implantat (42) eine drehbare Struktur (46) umfasst, die mit einem flexiblen Längselement (74) gekoppelt ist,
die drehbare Struktur (46) bidirektional drehbar ist, um einen Spannungsgrad des flexiblen Längselements (74) einzustellen, und
die zumindest eine Anschlussstation (56) dafür ausgelegt ist, die drehbare Struktur (46) aufzunehmen und mit dieser gekoppelt zu werden.

## Revendications

1. Dispositif destiné à l'utilisation d'au moins un implant (42), lequel comprend:
un élément de mise en prise avec du tissu pourvu a) d'une partie distale qui est conçue pour entrer en prise avec au moins une partie de tissu d'un patient et b) d'une partie proximale ;
au moins une station de raccordement (56) qui est couplée à la partie proximale de l'élément de mise en prise avec du tissu, ladite au moins une station de raccordement (56) :
étant conçue pour recevoir ledit au moins un implant (42) et être couplée à celui-ci et
comprenant un mécanisme de blocage (57) qui est conçu pour bloquer l'implant (42) au niveau de la station de raccordement (56) ; et
au moins un élément de guidage (40) qui est couplé de manière réversible à ladite au moins une station de raccordement (56), ledit au moins un élément de guidage (40) étant conçu pour faciliter une progression par glissement dudit au moins un implant (42) en direction de la station de raccordement (56).

2. Dispositif selon la revendication 1, dans lequel ladite au moins une station de raccordement (56) comprend deux stations de raccordement ou plus, et dans lequel ledit au moins un élément de guidage (40) comprend deux éléments de guidage ou plus, chaque élément de guidage (40) étant couplé de manière réversible à une station de raccordement (56) correspondante.

3. Dispositif selon la revendication 1, dans lequel l'implant (42) comprend au moins un implant qui est choisi parmi le groupe constitué par une valve cardiaque prothétique et un support destiné à recevoir une valve cardiaque prothétique, et dans lequel ladite au moins une station de raccordement (56) est conçue pour recevoir l'implant (42) sélectionné et être couplée à celui-ci.

4. Dispositif selon la revendication 1, dans lequel l'implant (42) comprend au moins un dispositif d'ajustage de tissu qui est choisi parmi le groupe constitué par un anneau d'annuloplastie partiel et un anneau d'annuloplastie complet, et dans lequel ladite au moins une station de raccordement (56) est conçue pour recevoir le dispositif d'ajustage de tissu sélectionné et être couplée à celui-ci.

5. Dispositif selon l'une quelconque des revendications 1 à 4, lequel comprend en outre l'implant (42).

6. Dispositif selon la revendication 5, dans lequel l'implant (42) présente:
une face supérieure (150) et une face inférieure (152),
au moins une première ouverture située sur la face supérieure (150),
au moins une deuxième ouverture située sur la face inférieure (152) et
un canal qui s'étend entre la première et la deuxième ouverture, le canal facilitant une progression par glissement de l'implant le long de l'élément de guidage (40).

7. Dispositif selon la revendication 6, dans lequel l'implant (42) présente un premier élément de couplage et dans lequel le mécanisme de blocage (57) présente un deuxième élément de couplage qui est conçu pour être couplé au premier élément de couplage.

8. Dispositif selon la revendication 7, dans lequel le deuxième élément de couplage (57) comprend au moins une partie en retrait, et dans lequel le premier élément de couplage comprend au moins une plaque de déviation mobile (47) qui est conçue pour entrer en prise avec au moins une partie en retrait du deuxième élément de couplage.

9. Dispositif selon l'une quelconque des revendications 1 à 2, lequel comprend en outre au moins un élément longitudinal flexible (74) qui est couplé à l'implant (42) au niveau d'une première partie, une deuxième partie de l'élément longitudinal flexible (74) étant conçue pour être couplée à une deuxième partie de tissu du patient et l'implant (42) étant conçu pour ajuster une longueur de l'élément longitudinal (74) située entre la première et la deuxième partie de tissu.

10. Dispositif selon la revendication 9, dans lequel:
la première partie de tissu comprend une première partie de tissu cardiaque située sur un premier site intraventriculaire (5),
la deuxième partie de tissu comprend au moins un feuillet (12, 14) d'une valve auriculo-ventriculaire du patient et
l'élément longitudinal flexible (74) comprend au moins une corde tendineuse artificielle.

11. Dispositif selon la revendication 9, dans lequel:
l'implant (42) comprend une structure rotative (46),
ledit au moins un élément longitudinal flexible (74) est couplé à la structure rotative (46) au niveau de la première partie et
la structure rotative (46) peut tourner de manière bidirectionnelle afin de régler le degré de tension dudit au moins un élément longitudinal flexible (74).

12. Dispositif selon la revendication 11, dans lequel la structure rotative (46) est conçue de telle sorte que:
une rotation de la structure rotative (46) dans un premier sens de rotation permet d'exercer une tension sur l'élément longitudinal flexible (74) et
une rotation de la structure rotative (46) dans un deuxième sens de rotation qui est opposé au premier sens de rotation permet d'atténuer la tension exercée sur l'élément longitudinal flexible (74).

13. Dispositif selon la revendication 11, lequel comprend en outre un mécanisme de blocage (45) de la structure rotative lequel peut être déplacé par rapport à ladite structure rotative (46) de telle sorte que celui-ci:
libère la structure rotative (46) pendant la rotation de la structure rotative (46) et
bloque la structure rotative (46) là où elle se trouve après la rotation de ladite structure rotative (46).

14. Dispositif selon la revendication 11, dans lequel la structure rotative (46) comprend une bobine (46) et dans lequel ledit au moins un élément longitudinal flexible (74) est conçu pour être enroulé autour de la bobine (46) pendant que ladite bobine (46) est en rotation dans un premier sens de rotation.

15. Dispositif selon l'une quelconque des revendications 1 à 4, dans lequel:
l'implant (42) comprend une structure rotative (46) qui est couplée à un élément longitudinal flexible (74),
la structure rotative (46) peut tourner de manière bidirectionnelle afin de régler un degré de tension de l'élément longitudinal flexible (74) et
ladite au moins une station de raccordement (56) est conçue pour recevoir la structure rotative (46) et être couplée à celle-ci.
